# EUROPÄISCHE PATENTSCHRIFT

(11) **EP 4 007 628 B1**
(45) Veröffentlichungstag und Bekanntmachung des Hinweises auf die Patenterteilung: **01.11.2023**
(21) Anmeldenummer: 20749843.7
(22) Anmeldetag: 28.07.2020
(51) Int. Cl.: A61M 16/00, A61M 16/20, A61M 16/08, A61M 16/12

(54) **BIDIREKTIONAL DURCHSTRÖMBARE ATEMGAS-VENTILBAUGRUPPE UND BEATMUNGSVORRICHTUNG MIT EINER SOLCHEN**
RESPIRATORY GAS VALVE ASSEMBLY THROUGH WHICH RESPIRATORY GAS CAN FLOW IN A BIDIRECTIONAL MANNER, AND VENTILATION DEVICE COMPRISING SUCH A VALVE ASSEMBLY
ENSEMBLE DE VALVE DE GAZ RESPIRATOIRE À TRAVERS LEQUEL UN GAZ RESPIRATOIRE PEUT S'ÉCOULER DE MANIÈRE BIDIRECTIONNELLE, ET DISPOSITIF DE VENTILATION COMPRENANT UN TEL ENSEMBLE DE VALVE

(30) Priorität: 01.08.2019 DE 102019120869; 12.08.2019 DE 102019121712
(43) Veröffentlichungstag der Anmeldung: 08.06.2022
(73) Patentinhaber: Hamilton Medical AG, 7402 Bonaduz (CH)
(72) Erfinder: LIPINSKI, Kerstin, 7014 Trin (CH); DENNEHY, Michael, 7403 Rhäzüns (CH); HALTINER, Kim, 7000 Chur (CH)
(74) Vertreter: Ruttensperger Lachnit Trossin Gomoll
(86) Internationale Anmeldenummer: PCT/EP2020/071292
(87) Internationale Veröffentlichungsnummer: WO 2021/018902

(56) Entgegenhaltungen:
- DE-A1-102004 011 907
- DE-C1- 4 345 123
- US-A- 5 050 593
- US-A1- 2011 197 889
- US-A1- 2013 267 863
- US-A1- 2015 101 610
- US-B1- 6 427 692

## Beschreibung

Die vorliegende Erfindung betrifft eine Atemgas-Ventilbaugruppe für eine Beatmungsleitungsanordnung einer Beatmungsvorrichtung zur wenigstens unterstützenden künstlichen Beatmung eines Patienten gemäß dem Oberbegriff des Anspruchs 1. Die vorliegende Erfindung betrifft außerdem eine Beatmungsvorrichtung mit einer solchen Atemgas-Ventilbaugruppe, wobei die Atemgas-Ventilbaugruppe wenigstens zusätzlich zu einem an sich bekannten Exspirationsventil vorgesehen ist.

Eine gattungsgemäße Atemgas-Ventilbaugruppe ist aus der US 6,427,692 B1 bekannt.

Aus der US 2011/0197889 A1 ist eine Beatmungsvorrichtung mit baulich getrennten Atemgas führenden Leitungen, nämlich einer Inspirationsleitung und einer Exspirationsleitung bekannt. Jede der baulich getrennten Leitungen weist ein gesondertes Rückschlagventil auf, welches Atemgas nur in die für die jeweilige Leitung bestimmte Richtung durchlässt und in entgegengesetzter Richtung sperrt.

Aus der US 5,050,593 ist eine weitere Beatmungsvorrichtung bekannt, welche in baulich getrennten Inspirations- und Exspirationsleitungen baulich getrennte und jeweils konstruktiv unterschiedlich aufgebaute Ventile auf, welche Atemgas nur in die für die jeweilige Leitung vorgesehene Richtung durchlassen. Dabei ist das Ventil in der Inspirationsleitung ohne weiteres geöffnet und wird mittels eines Bypass-Schlauchs durch Atemgas in der Exspirationsleitung während einer Exspirationsphase geschlossen.

Aus der US 2007/0193581 A1 ist eine Beatmungsvorrichtung bekannt, umfassend ein Beatmungsgerät mit einer Atemgasquelle, eine proximal angeordnete Spirometrie-Sensorbaugruppe, eine Patientenschnittstelle zur Abgabe von inspiratorischem Atemgas an einen Patienten und zur Aufnahme von exspiratorischem Atemgas vom Patienten, sowie eine die Beatmungsvorrichtung mit der Spirometrie-Sensorbaugruppe verbindende Beatmungsleitungsanordnung. Die aus der US 2007/0193581 A1 bekannte Beatmungsleitungsanordnung ist eine koaxiale Beatmungsleitungsanordnung, bei welcher die Inspirationsleitung im Inneren der Exspirationsleitung koaxial zu dieser verlaufend angeordnet ist. In dem bekannten Beispiel ist die Exspirationsleitung folglich als ein die Inspirationsleitung umgebender Ringkanal ausgebildet. Hierdurch kann ein zur Beatmungsleitungsanordnung beitragender Schlauch, obwohl er einen exspiratorischen und einen inspiratorischen Strömungspfad aufweist, nur einen einzigen von außen greifbaren Schlauch aufweisen. Diese kompakte Bauweise verhindert ein unerwünschtes Verheddern einer Pflegeperson in gesonderten inspiratorischen und exspiratorischen Schläuchen.

Von der proximal, also patientennah, angeordneten Spirometrie-Sensorbaugruppe gehen weitere Leitungen bzw. Schläuche ab, die zum Beatmungsgerät und der darin ebenfalls vorgesehenen Auswerte- und Steuervorrichtung verlaufen. Zur Vermeidung von unerwünschtem Schlauchgewirr unter Beteiligung der von der Sensorbaugruppe abgehenden Schläuche sind diese innerhalb der Atemgas führenden Schläuche verlegt. Dabei können alle Schläuche nur in dem inneren inspiratorischen Schlauch oder nur in dem äußeren exspiratorischen Schlauch verlaufen oder können auf beide Schläuche aufgeteilt verlegt sein.

Aus der EP 3 090 774 A1 ist ein Bi-Lumen-Atemgasschlauch bekannt, welcher ebenfalls einen inspiratorischen und einen exspiratorischen Strömungspfad aufweist. Die Lumina dieses bekannten Bi-Lumen-Atemgasschlauchs sind abweichend von der koaxialen Beatmungsleitungsanordnung der US 2007/0193581 A1 durch ein Septum körperlich voneinander getrennt, welches zum einen in Längsrichtung des Schlauchs und zum anderen orthogonal zur Längsrichtung des Schlauchs zwischen Schlauchwandabschnitten verläuft, welche einander bezüglich einer den Schlauch in dessen Längsrichtung zentral durchsetzend gedachten virtuellen Schlauchbahn gegenüberliegen. Aus der EP 3 090 774 A1 ist es außerdem bekannt, eine Nebenstromleitung, durch welche eine Menge an Atemgas aus dem Atemgas-Hauptstrom zur Nebenstrom-Messung des CO₂-Gehalts abgezweigt wird, durch eines der beiden Lumina des Bi-Lumen-Atemgasschlauchs zu verlegen.

Das Exspirationsventil der Beatmungsvorrichtung der US 2007/019358 A1 befindet sich distal, also patientenfern, am Beatmungsgerät. Wechselt das Beatmungsgerät von einer Exspirationsphase zu einer Inspirationsphase muss das Exspirationsventil des Beatmungsgeräts geschlossen und ein üblicherweise am Beatmungsgerät angeordnetes Inspirationsventil geöffnet werden, damit der Patient sicher inspiratorisches Atemgas in der benötigten Menge in der zur Verfügung stehenden Zeit erhält. Sofern eine eigene Ventilsteuerung zur aktiven Betätigung des Exspirationsventils und des Inspirationsventils vorgesehen ist, ist der Beatmungsbetrieb weitgehend unkritisch. An robust, aber dafür einfach aufgebauten Notfall-Beatmungsvorrichtungen kann es dagegen zu Schwierigkeiten kommen.

Notfall-Beatmungsvorrichtungen weisen häufig ein Exspirationsventil auf, welches nicht durch einen eigenen Aktuator, sondern durch den Druck des inspiratorischen Atemgases während einer Inspirationsphase geschlossen wird bzw. geschlossen gehalten wird. Häufig wird bei Notfall-Beatmungsvorrichtungen der notwendige Druck im inspiratorischen Atemgas zu Beginn einer Inspirationsphase nicht schnell genug aufgebaut, sodass das Exspirationsventil nicht ausreichend schnell und sicher geschlossen wird. In der Folge kann der Patient unerwünschterweise bereits ausgeatmetes Atemgas aus dem exspiratorischen Leitungsbereich zwischen der Patientenschnittstelle und dem Exspirationsventil erneut einatmen, was zu einer Unterversorgung des Patienten mit dem inspiratorischen Atemgas führen kann.

Außerdem ist im Falle einer distalen Anordnung des Exspirationsventils die verhältnismäßig lange Gassäule zwischen Patient und Exspirationsventil als eine Art Gasfeder schwingungsfähig und kann leicht zu Schwingungen angeregt werden, die einen Schließzustand des Exspirationsventils gefährden, sodass das Exspirationsventil nicht wirklich geschlossen ist oder bleibt, obwohl es aufgrund der Steuerbefehle des Beatmungsgeräts geschlossen sein sollte.

Aufgabe der vorliegenden Erfindung ist es daher, eine technische Lehre anzugeben, welche es ermöglicht, den Patienten sicherer als oben dargestellt in den von einem Beatmungsgerät vorgegebenen Beatmungszyklen mit den vorgesehenen Atemgasmengen in der gewünschten Atemgaszusammensetzung zu versorgen.

Diese Aufgabe löst die vorliegende Erfindung durch eine Atemgas-Ventilbaugruppe für eine Beatmungsleitungsanordnung einer Beatmungsvorrichtung zur wenigstens unterstützenden künstlichen Beatmung eines Patienten mit allen Merkmalen des Anspruchs 1. Gemäß einer erfindungsgemäßen Ausführungsform weist die Atemgas-Ventilbaugruppe ein Gehäuse mit einer bidirektional von Atemgas durchströmbaren Kanalanordnung auf. Die Kanalanordnung weist einen inspiratorischen und einen exspiratorischen Strömungspfad auf, wobei der inspiratorische Strömungspfad der Kanalanordnung von einem distalen Einlassendbereich zu einem proximalen Auslassendbereich der Atemgas-Ventilbaugruppe verläuft und wobei der exspiratorische Strömungspfad der Kanalanordnung von einem proximalen Einlassendbereich zu einem distalen Auslassendbereich der Atemgas-Ventilbaugruppe verläuft. Die Atemgas-Ventilbaugruppe weist dabei zwischen ihren jeweiligen Einlass- und Auslassendbereichen eine Ventilanordnung auf, welche wenigstens verstellbar ist zwischen einer ersten Betriebsstellung, in welcher der inspiratorische Strömungspfad zur

Durchströmung freigegeben und der exspiratorische Strömungspfad für eine Durchströmung gesperrt ist, und einer zweiten Betriebsstellung, in welcher der exspiratorische Strömungspfad zur Durchströmung freigegeben und der inspiratorische Strömungspfad für eine Durchströmung gesperrt ist. Dabei verlaufen der inspiratorische und der exspiratorische Strömungspfad auf wenigstens einer baulichen Seite der Atemgas-Ventilbaugruppe bezüglich der Ventilanordnung in körperlich voneinander getrennten Strömungsführungsabschnitten der Kanalanordnung.

Die vorgeschlagene Atemgas-Ventilbaugruppe kann proximal, etwa in Inspirationsrichtung stromabwärts eines von einem Beatmungsgerät mit Druckveränderungsvorrichtung zur Veränderung eines Drucks des Atemgases weg verlaufenden Beatmungsschlauchs, vorgesehen sein, sodass sie nahe des Patienten eine Atemgasströmung im Beatmungsschlauch steuern kann. Durch die genannten Betriebsstellungen kann die Atemgas-Ventilbaugruppe die Gefahr erheblich reduzieren, dass der Patient bereits ausgeatmetes Atemgas erneut einatmet.

Dies leistet die Atemgas-Ventilbaugruppe, die nachfolgend auch nur kurz als "Ventilbaugruppe" bezeichnet ist, zum einen dadurch, dass sie ermöglicht, während einer Inspirationsphase einen großen Teil des exspiratorischen Strömungspfads, nämlich vom Exspirationsventil bis zur Ventilanordnung der Ventilbaugruppe, für eine Durchströmung zu sperren. Der Patient kann dann Atemgas nur durch den inspiratorischen Strömungspfad erhalten, wie es bestimmungsgemäß gewünscht ist.

Dies leistet die Ventilbaugruppe zum anderen dadurch, dass sie ermöglicht, während einer Exspirationsphase den inspiratorischen Strömungspfad für eine Durchströmung zu sperren. Somit kann der Patient während einer Exspirationsphase kein exspiratorisches Atemgas, oder erheblich weniger exspiratorisches Atemgas als im Stand der Technik möglich, in den inspiratorischen Strömungspfad ausatmen, welches er in der nachfolgenden Inspirationsphase ansonsten erneut verabreicht bekäme.

Durch die Möglichkeit zur proximalen Anordnung der Ventilbaugruppe wird eine Schwingungsanregung einer Gassäule in einem in Exspirationsrichtung stromabwärts der Ventilbaugruppe bzw. der Ventilanordnung gelegenen Atemgas-Leitungsabschnitt erheblich erschwert. Denn die Ventilanordnung ermöglicht, die Gassäule in dem in der jeweiligen Beatmungsphase: Inspirationsphase und Exspirationsphase, nicht benötigten Strömungspfad nahe des Patienten pneumatisch von diesem zu entkoppeln.

Mit der Nennung einer "baulichen Seite" der Ventilbaugruppe bezüglich der Ventilanordnung soll sprachlich von einer funktionalen Seite der Ventilbaugruppe bezüglich der Ventilanordnung unterschieden werden. So liegen die funktional einander entsprechenden Einlassendbereiche einmal für exspiratorisches und ein andermal für inspiratorisches Atemgas, die funktionsbedingt jeweils stromaufwärts der Ventilanordnung liegen müssen, üblicherweise auf baulich unterschiedlichen Seiten der Ventilbaugruppe bezüglich der Ventilanordnung. Entsprechendes gilt für die Auslassendbereiche.

Grundsätzlich kann auf beiden baulichen Seiten der Ventilbaugruppe bezüglich der Ventilanordnung im Gehäuse der Ventilbaugruppe der inspiratorische und der exspiratorische Strömungspfad voneinander körperlich getrennt in jeweils gesonderten Kanallumina ausgebildet sein.

Häufig ist ein proximaler Abschnitt einer Atemgas vom Beatmungsgerät bis zum Patienten leitenden Beatmungsleitungsanordnung mit einem gemeinsamen sowohl exspiratorisch als auch inspiratorisch verwendeten Kanallumen ausgebildet. Dieser Übergang zwischen gesondert und gemeinsam ausgebildeten Strömungspfaden für Inspiration und für Exspiration kann vorteilhaft in der Ventilbaugruppe realisiert sein. Hierzu können der inspiratorische und der exspiratorische Strömungspfad auf der einen baulichen Seite der Ventilbaugruppe bezüglich der Ventilanordnung in körperlich voneinander getrennten Kanalabschnitten mit baulich getrennten Kanallumina verlaufen und können auf der anderen baulichen Seite der Ventilbaugruppe bezüglich der Ventilanordnung in einem gemeinsamen Strömungsführungsabschnitt der Kanalanordnung und somit in einem gemeinsamen Kanallumen verlaufen. Somit kann zum einen die vorteilhafte Steuerungswirkung der Ventilbaugruppe und zum anderen ein möglichst kleines patientenseitiges Totvolumen in der Beatmungsleitungsanordnung erhalten werden. Bevorzugt umfasst die bauliche Seite mit den körperlich voneinander getrennten Kanalabschnitten den distalen Einlassendbereich für inspiratorisches Atemgas und den distalen Auslassendbereich für exspiratorisches Atemgas. Die bauliche Seite mit dem gemeinsamen Strömungsführungsabschnitt umfasst folglich bevorzugt den proximalen Einlassendbereich für exspiratorisches Atemgas und den proximalen Auslassendbereich für inspiratorisches Atemgas.

Grundsätzlich kann die Ventilbaugruppe als Y-Verbinder ausgebildet sein, sodass distal an das Gehäuse der Ventilbaugruppe ein inspiratorischer und ein exspiratorischer Beatmungsschlauch anschließbar sind und sodass proximal ein gemeinsamer Leitungsabschnitt für Inspiration und Exspiration anschließbar ist. Zur Verringerung der Anzahl an von außen für Pflege- und insbesondere für Rettungspersonal greifbaren Beatmungsschläuchen und damit zur Vermeidung von unerwünschtem "Schlauchsalat" ist es bevorzugt, wenn die körperlich voneinander getrennten Kanalabschnitte auf der einen baulichen Seite der Ventilbaugruppe bezüglich der Ventilanordnung durch einen Mehrlumenkanal gebildet sind. Der Mehrlumenkanal kann ein Kanal mit konzentrischen Lumina sein. Wegen der nicht nur betragsmäßig hinsichtlich der Querschnittsfläche, sondern auch hinsichtlich der Querschnittsgestalt gleichen oder wenigstens ähnlichen Lumina ist jedoch ein Kanal mit orthogonal zur Kanallängsrichtung nebeneinander verlaufenden Lumina bevorzugt. Die Lumina sind dann durch ein sowohl in Kanallängsrichtung als auch orthogonal dazu verlaufendes Septum voneinander getrennt, welches eine gemeinsame Trennwand für wenigstens zwei aneinander angrenzende Lumina bildet. Außerhalb des Septums sind zwei aneinander angrenzende Lumina durch jeweils eine Kanalwand umgrenzt, die keine gemeinsame Trennwand zwischen den beiden Lumina ist. Nur das Septum wird also beiderseits von Atemgas benetzt, die übrigen Kanalwandabschnitte dagegen nicht.

Zur Erleichterung des Anschlusses einer Leitungskomponente, insbesondere eines Beatmungsschlauchs, an den distalen Endbereich der Ventilbaugruppe kann gemäß einer vorteilhaften Weiterbildung der vorliegenden Erfindung vorgesehen sein, dass ein sowohl den distalen Einlassendbereich als auch den distalen Auslassendbereich aufweisender distaler Endbereich der Ventilbaugruppe als distale Anschlussformation zum Anschluss einer distalen Leitungskomponente ausgebildet ist. Bevorzugt ist die distale Leitungskomponente ein Mehr-Lumen-Beatmungsschlauch, wobei in der Regel ein Bi-Lumen-Beatmungsschlauch mit lediglich zwei Lumina ausreicht, um die erforderlichen Funktionen Inspiration und Exspiration abzubilden. Die Anschlussformation kann unter anderem eine Schnellkupplung sein oder kann eine Buchse oder ein Stecker einer Steckverbindung sein.

Aus dem gleichen Grunde einer möglichst schnellen und einfachen Verbindbarkeit der Ventilbaugruppe an ihrem proximalen Endbereich mit einer weiteren, zum Patienten führenden Leitungskomponente kann vorgesehen sein, dass ein sowohl den proximalen Einlassendbereich als auch den proximalen Auslassendbereich aufweisender proximaler Endbereich der Ventilbaugruppe als proximale Anschlussformation zum Anschluss einer proximalen Leitungskomponente ausgebildet ist. Eine solche proximale Leitungskomponente kann ein proximaler Beatmungsschlauch, dann bevorzugt als Mono-Lumen-Beatmungsschlauch, oder ein Atemgassensor, wie etwa ein Durchflusssensor oder eine Messküvette für eine Ermittlung von Anteilen an Gasbestandteilen des Atemgases, sein. Die Messküvette kann mit einem nichtdispersiven CO₂- oder/und NO₂-Infrarotsensor oder/und mit einem fluoreszenzspektroskopischen O2-Sensor zusammenwirken.

Ein an die Ventilbaugruppe als Teil der Beatmungsleitungsanordnung einer Beatmungsvorrichtung angeschlossener Atemgassensor kann weitere Leitungen erfordern, um Sensorinformation vom Atemgassensor an eine Steuervorrichtung der Beatmungsvorrichtung zu übertragen. Zur Vermeidung eines durch die weiteren Leitungen bewirkten unerwünschten Leitungsgewirrs kann die Atemgas-Ventilbaugruppe eine Leitungsanordnung aufweisen, welche mit wenigstens einem körperlich von der Atemgas führenden Kanalanordnung getrennten Lumen ausgebildet ist, wobei ein innerer Teil der Leitungsanordnung derart innerhalb der Kanalanordnung verläuft, dass wenigstens ein Abschnitt einer Außenseite des inneren Teils der Leitungsanordnung im Betrieb von Atemgas benetzbar ist, oder/und wobei ein äußerer Teil der Leitungsanordnung derart außerhalb eines von Atemgas durchströmbaren Kanalvolumens der Kanalanordnung verläuft, dass der äußere Teil der Leitungsanordnung im Betrieb von Atemgas nicht benetzbar ist. Auf diese Weise kann die Leitungsanordnung mit ihrem äußeren Teil, der sich außerhalb des Gehäuses der Ventilbaugruppe befindet, an einen Atemgassensor oder an ein anderes Bauteil der Beatmungsleitungsanordnung angeschlossen werden. Mit ihrem inneren Teil verläuft die Leitungsanordnung von außen durch das Gehäuse der Ventilbaugruppe verdeckt, sodass die Leitungsanordnung im Inneren einer an den jeweiligen Endbereich, vorzugsweise den distalen Endbereich, der Ventilbaugruppe angeschlossenen Leitungskomponente weitergeführt werden kann. Die Leitungsanordnung ist dann nur in dem, verglichen mit der Gesamtlänge der Leitungsanordnung, kurzen äußeren Teil von außen greifbar, der für den Anschluss der Leitungsanordnung an ein weiteres Bauteil benötigt wird.

Die Leitungsanordnung kann grundsätzlich eine elektrische Leitungsanordnung oder/und eine pneumatische Leitungsanordnung sein. Eine elektrische Leitungsanordnung, umfassend eine Isolation, in deren Lumen elektrisch leitfähige Drähte oder Litzen geführt sind, kann elektrische Signale von dem oben genannten InfrarotSensor oder/und dem oben genannten fluoreszenzspektroskopischen Sensor zu einer Steuervorrichtung im oder am Beatmungsgerät übertragen. Eine pneumatische Leitungsanordnung kann einen distalen und einen proximalen Atemgasdruck beiderseits eines veränderlichen Strömungswiderstands eines Differenzdruck-Durchflusssensors zu Drucksensoren im oder am Beatmungsgerät übertragen. Die Leitungsanordnung kann daher eine oder mehrere Leitungen bzw. eine Mono- oder Mehr-Lumen-Leitung umfassen.

Um zu vermeiden, dass der innere Teil der Leitungsanordnung an der Ventilanordnung im Gehäuse der Ventilbaugruppe vorbeigeführt werden muss, ist es bevorzugt, dass der innere Teil der Leitungsanordnung und wenigstens ein Abschnitt des äußeren Teils der Leitungsanordnung zwischen den distalen Endbereichen der Atemgas-Ventilbaugruppe und der Ventilanordnung ausgebildet sind. Hierzu kann die Leitungsanordnung das Gehäuse der Atemgas-Ventilbaugruppe zwischen den distalen Endbereichen der Atemgas-Ventilbaugruppe und der Ventilanordnung durchsetzen.

Die Leitungsanordnung kann mehrstückig ausgebildet sein. Beispielsweise kann sie eine einstückig mit dem Ventilgehäuse ausgebildete oder an das Ventilgehäuse montierte äußere Anschlussstutzenanordnung aufweisen, welche aus dem Ventilgehäuse nach außen herausragt. An die äußere Anschlussstutzenanordnung ist eine flexible Schlauchanordnung als weiterer äußerer Teil der Leitungsanordnung anschließbar.

Ebenso kann die Leitungsanordnung eine einstückig mit dem Ventilgehäuse ausgebildete oder an das Ventilgehäuse montierte innere Anschlussstutzenanordnung aufweisen, welche in das Ventilgehäuse nach innen hineinragt. An die innere Anschlussstutzenanordnung ist eine flexible Schlauchanordnung als weiterer innerer Teil der Leitungsanordnung anschließbar. Die letztgenannte flexible Schlauchanordnung kann eine Schlauchanordnung sein, welche in einem Beatmungsschlauch, insbesondere in einem Mehr-Lumen-Schlauch, wenigstens längsabschnittsweise innerhalb eines Lumens des Beatmungsschlauchs aufgenommen und geführt ist.

Insbesondere der innere Teil der Leitungsanordnung kann einstückig mit dem Gehäuse oder einem Gehäusebauteil des Gehäuses der Ventilbaugruppe hergestellt sein, beispielsweise durch Spritzgießen. Hier können Gas- oder Projektil-Injektions-Techniken bei der spritzgusstechnischen Herstellung des Gehäuses oder Gehäusebauteils mit dem einstückig daran ausgeformten inneren Teil der Leitungsanordnung verwendet werden. Selbstverständlich kann zusätzlich oder alternativ der äußere Teil der Leitungsanordnung einstückig mit dem Gehäuse oder einem Gehäusebauteil der Ventilbaugruppe ausgebildet sein.

Das Gehäuse der Ventilbaugruppe kann wenigstens zwei gesondert voneinander ausgebildete Gehäusebauteile umfassen, welche miteinander zu dem Gehäuse der Ventilbaugruppe verbindbar, insbesondere verrastbar, sind. Wenigstens ein Teil der Ventilanordnung, insbesondere die Ventilkörper der Ventilanordnung, kann zwischen den Gehäusebauteilen angeordnet sein und durch Verbindung der Gehäusebauteile miteinander zwischen diesen geklemmt gehalten sein. Wenigstens eines der Gehäusebauteile kann den zwischen den Gehäusebauteilen gehaltenen Teil der Ventilanordnung körperlich wenigstens abschnittsweise umgreifen und den Teil so zusätzlich zu einer klemmenden oder reibschlüssigen Fixierung körperlich festlegen.

Erfindungsgemäß weist die Ventilanordnung eine Ventilkörperanordnung auf, umfassend einen Ventilkörperrahmen und am Ventilkörperrahmen beweglich gehalterte Ventilkörper, insbesondere Ventilkörperblätter, zur Bildung von Blattventilen. Der Ventilkörperrahmen kann zwischen zwei Gehäusebauteilen eingeklemmt sein und die Ventilkörperblätter können dadurch in jeweiligen Strömungskanälen im Inneren der Gehäusebauteile zwischen einer Schließstellung und einer Öffnungsstellung beweglich sein. Eine solche Ventilkörperanordnung kann einstückig als, vorzugsweise ebene, Ventilkörperanordnung mit zwei Blattventilen, eines für jeden Strömungspfad aus inspiratorischem und exspiratorischem Strömungspfad, ausgebildet sein.

Die innere und die äußere Anschlussstutzenanordnung können einstückig an einem Rohrstück ausgebildet sein. Dabei kann ein Rohrstück für jede Leitung der Leitungsanordnung vorgesehen sein. Das Rohrstück kann in eine Ausnehmung am Gehäuse der Ventilanordnung einsetzbar sein oder kann einstückig mit dem Gehäuse ausgebildet sein. Bevorzugt erstreckt sich das Rohrstück mit seiner vorzugsweise geradlinigen Rohrachse parallel zum Verlauf des inspiratorischen oder/und des exspiratorischen Strömungspfads am baulich distalen Endbereich des Gehäuses, sodass die im Beatmungsschlauch geführte Leitungsanordnung, welche im Beatmungsschlauch vorzugsweise stets parallel zum inspiratorischen oder/und exspiratorischen Strömungspfad verläuft, ohne zusätzliche Umlenkung in möglichst geradlinigem Verlauf durch das Gehäuse der Ventilanordnung nach außen geführt sein kann, wo die Leitungsanordnung über die äußere Anschlussstutzenanordnung mit einer weiteren Funktionsbaugruppe, beispielsweise einer biometrischen Funktionsbaugruppe, insbesondere einem Differenzdruck-Durchflusssensor, verbunden werden kann. Durch die Vermeidung einer Umlenkung der Leitungsanordnung wird eine mechanische Belastung desselben vermieden. Sofern die Leitungsanordnung eine pneumatische Leitungsanordnung ist, wird durch die Vermeidung einer Umlenkung der von ihr bewirkte Strömungswiderstand verringert, was insbesondere die Erfassungsgenauigkeit eines angeschlossenen Differenzdruck-Durchflusssensors erhöht.

Alternativ kann die Leitungsanordnung eine flexible Schlauchanordnung umfassen, welche eine Öffnungsanordnung im Ventilgehäuse in Dickenrichtung des Ventilgehäuses vollständig durchsetzt. Die Öffnungsanordnung kann radial innen mit Dichtmitteln, etwa mit einer oder mehreren umlaufenden Elastomerdichtungen, insbesondere Silikongummidichtungen, versehen sein, welche radial außen an der durchsetzenden Schlauchanordnung anliegen. Die Schlauchanordnung kann eine Mehrzahl von gesondert ausgebildeten flexiblen Schläuchen aufweisen, welche funktional parallel oder/und in Reihe zueinander verlaufen. Alternativ kann die Schlauchanordnung ein Schlauch mit mehreren Lumina sein. Diese Schlauchanordnung kann mehrere in Schlauchanordnungs-Längsrichtung aufeinander folgende gesonderte Abschnitte aufweisen, etwa wenn die Durchsetzungsstellen am Ventilgehäuse als Anschlussstutzenanordnungen ausgebildet sind.

Grundsätzlich kann es ausreichen, wenn die Ventilanordnung nur ein die Durchströmbarkeit des exspiratorischen Strömungspfads veränderndes exspiratorisches Ventil aufweist. Dann kann zumindest verhindert werden, dass nach einer Exspirationsphase in einem unerwünscht großen Totraum des exspiratorischen Strömungspfads vorhandenes exspiratorisches Atemgas während einer nachfolgenden Inspirationsphase erneut eingeatmet wird. Auch eine Schwingungsanregung einer Gassäule im exspiratorischen Strömungspfad kann so reduziert werden.

Bevorzugt soll jedoch darüber hinaus auch dafür gesorgt werden, dass exspiratorisches Atemgas nicht oder zumindest nicht weit längs des inspiratorischen Strömungspfads strömt und von dort während einer nachfolgenden Inspirationsphase erneut dem Patienten zugeführt wird. Daher ist erfindungsgemäß vorgesehen, dass die Ventilanordnung ein die Durchströmbarkeit des inspiratorischen Strömungspfads steuerndes inspiratorisches Ventil und ein die Durchströmbarkeit des exspiratorischen Strömungspfads steuerndes exspiratorisches Ventil aufweist. Durch Vorsehen des inspiratorischen Ventils in der Ventilanordnung der Ventilbaugruppe kann die vorliegende Atemgas-Ventilbaugruppe in einer sehr einfach, aber sehr robust aufgebauten Notfall-Beatmungsvorrichtung eingesetzt werden. In dieser Notfall-Beatmungsvorrichtung, wie sie als portable Beatmungsvorrichtung von Rettungsdiensten, Notärzten usw. in Rettungsfahrzeugen und Rettungsflugzeugen, eingesetzt wird, kann das inspiratorische Ventil der Ventilanordnung das einzige im inspiratorischen Strömungspfad angeordnete Ventil sein. Ein üblicherweise an einem Beatmungsgerät distal vorgesehenes Inspirationsventil kann dann entfallen.

Weiter sind erfindungsgemäß das inspiratorische und das exspiratorische Ventil in seine den jeweiligen Strömungspfad gegen eine Durchströmung sperrende Sperrstellung vorgespannt, sodass es jeweils einer Aktion bedarf, um Atemgas längs eines der beiden Strömungspfade zu leiten. Erfindungsgemäß wird der Druck inspiratorischen Atemgases erhöht, um das inspiratorische Ventil gegen seine Vorspannung in die Sperrstellung zu öffnen und dem Patienten inspiratorisches Atemgas zuzuleiten. Ebenso strömt erfindungsgemäß nach einem Abklingen eines Überdrucks im inspiratorischen Atemgas exspiratorisches Atemgas mit Überdruck aus der Patientenlunge zurück, wobei der Überdruck des exspiratorischen Atemgases nicht nur eine zusätzliche Schließkraft auf das inspiratorische Ventil ausübt, sondern gleichzeitig das exspiratorische Ventil gegen seine Vorspannung in die Sperrstellung öffnet. Erfindungsgemäß sind beide Ventile in ihre Sperrstellung vorgespannt. Es gibt dann eine dritte Betriebsstellung, in welcher beide Strömungspfade durch die Ventilanordnung gleichzeitig gegen eine Durchströmung gesperrt sind.

Zur Vereinfachung des konstruktiven Aufbaus reicht es dabei gemäß einer vorteilhaften Weiterbildung aus, wenn das inspiratorische oder/und das exspiratorische Ventil jeweils als Rückschlagventil ausgebildet ist bzw. sind. Ein einfaches Rückschlagventil mit einem in kurzer Zeit herstellbaren großen Durchlassquerschnitt ist ein Rückschlag-Klappenventil, vorzugsweise in Gestalt des zuvor genannten Blattventils mit einem durch Biegung aus der Schließstellung auslenkbaren Blatt als Ventilkörper. Ein solcher Ventilkörper ist in der vorliegenden Anmeldung auch als "Ventilkörperblatt" bezeichnet. Ein solches Rückschlag-Klappenventil kann um eine zum jeweiligen Strömungspfad orthogonal orientierte Klappen-Schwenkachse schwenkbar im Gehäuse aufgenommen sein. Eine komfortable schwenkbare Anordnung, vorzugsweise des inspiratorischen oder/und des exspiratorischen Rückschlag-Klappenventils, bietet sich im Bereich des Längsendes eines Kanalseptums an, welches auf einer baulichen Seite der Ventilbaugruppe bezüglich der Ventilanordnung die im Gehäuse ausgebildete Kanalanordnung in ein inspiratorisches und ein exspiratorisches Lumen trennt. Wie oben dargelegt wurde, endet das Septum bevorzugt an der Ventilanordnung, sodass an dem Ende des Septums im Gehäuse eine ebene, geradlinig verlaufende Montagefläche zur beweglichen Anordnung von Klappen als Ventilkörper des inspiratorischen und des exspiratorischen Rückschlagventils ausgebildet sein kann. Zusätzlich oder alternativ kann ein Klappen-Ventilkörper des inspiratorischen oder/und des exspiratorischen Rückschlag-Klappenventils schwenkbar an seinem Ventilsitz angeordnet sein.

Die vorliegende Erfindung betrifft außerdem eine Beatmungsvorrichtung zur wenigstens unterstützend künstlichen Beatmung eines lebenden Patienten, umfassend:
- eine Atemgasquelle,
- eine Beatmungsleitungsanordnung, um inspiratorisches Atemgas von der Atemgasquelle zu einer patientenseitigen, proximalen Atemgas-Auslassöffnung und um exspiratorisches Atemgas von einer proximalen Atemgas-Einlassöffnung weg zu leiten,
- eine Druckveränderungsvorrichtung zur Veränderung des Drucks des Atemgases in der Beatmungsleitungsanordnung sowie
- eine Steuervorrichtung zum Betrieb der Atemgasquelle oder/und der Druckveränderungsvorrichtung,
wobei die Beatmungsvorrichtung bevorzugt eine Atemgas-Ventilbaugruppe aufweist, wie sie oben beschrieben und weitergebildet ist.

Die Beatmungsleitungsanordnung bezeichnet die vollständige Leitungsanordnung zur Leitung inspiratorischen Atemgases von der Atemgasquelle bis zum Patienten sowie zur Leitung exspiratorischen Atemgases vom Patienten weg in die Umgebung bzw. in eine etwaig vorgesehene Atemgassenke.

Als Atemgasquelle kann ein Behälter mit einem Atemgasvorrat, eine Ansaugöffnung mit Gebläse zum Ansaugen von Luft aus der Umgebungsatmosphäre oder/und eine Anschlussformation zum Anschluss der Druckveränderungsvorrichtung und der Beatmungsleitungsanordnung an eine einen Atemgasvorrat umfassende Hausinstallation sein, wie sie in Kliniken häufig anzutreffen ist.

Die Druckveränderungsvorrichtung kann ein Gebläse oder/und ein Ventil umfassen.

Die Beatmungsvorrichtung ist bevorzugt eine nicht nur mobile, sondern tragbare Notfall-Beatmungsvorrichtung für medizinische Rettungseinsätze. Sie kann jedoch auch eine klinisch-stationäre Beatmungsvorrichtung sein. Bei der oben genannten bevorzugten Ausgestaltung der Ventilanordnung mit einem exspiratorischen und einem inspiratorischen Ventil im Gehäuse der Ventilbaugruppe weist die Beatmungsvorrichtung bevorzugt kein weiteres Inspirationsventil auf. Der Druck von inspiratorischem Atemgas kann dann während einer Inspirationsphase alleine durch ein Gebläse erhöht werden bzw. sein, wobei das Gebläse zur Exspiration entweder in seiner Leistung gedrosselt oder abgeschaltet wird.

Gerade für Notfall-Einsätze ist es vorteilhaft, wenn sich ein medizinisches Rettungsteam vollumfänglich auf seine medizinischen Aufgaben konzentrieren kann und sich nicht darum kümmern muss, sich nicht in Schläuchen der Beatmungsvorrichtung zu verheddern. Daher umfasst die Beatmungsleitungsanordnung bevorzugt einen Multi-Lumen-Schlauch als den oben genannten Beatmungsschlauch, wobei wegen der lediglich zwei benötigten entgegengesetzten Strömungspfade: inspiratorischer und exspiratorischer Strömungspfad, ein Bi-Lumen-Schlauch ausreicht. Der Multi-Lumen-Schlauch umfasst weiter eine wenigstens längs eines Längsabschnitts des Multi-Lumen-Schlauchs in wenigstens einem Lumen geführte Leitungsanordnung. Zu der Leitungsanordnung gilt das oben im Zusammenhang mit der Ventilbaugruppe Gesagte.

Bevorzugt ist zwischen dem proximalen Längsende des Beatmungsschlauchs einerseits und der proximalen Atemgas-Auslassöffnung oder/und der proximalen Atemgas-Einlassöffnung andererseits eine Spirometrie-Sensoranordnung angeordnet, mit welcher die Leitungsanordnung verbunden ist.

Weiter bevorzugt umfasst oder ist die Spirometrie-Sensoranordnung eine Durchflusssensoranordnung zur Erfassung des inspiratorischen oder/und des exspiratorischen Atemgasflusses. Besonders bevorzugt ist die Durchflusssensoranordnung eine Differenzdruck-Durchflusssensoranordnung mit einem veränderlichen Strömungswiderstand. Dann umfasst die Leitungsanordnung bevorzugt Gasdruckleitungen, welche die Atemgasdrücke beiderseits des Strömungswiderstands zum Zwecke ihrer Quantifizierung zu einem Drucksensor im Beatmungsgerät übertragen.

Als Alternative zur oben beschriebenen Durchsetzung des Gehäuses der Ventilbaugruppe durch die Leitungsanordnung kann daran gedacht sein, dass die Leitungsanordnung eine den Multi-Lumen-Schlauch zur Außenumgebung hin begrenzende Schlauchwand durchsetzt. Dadurch wird der Zusammenbau der Beatmungsvorrichtung bzw. ein aus welchen Gründen auch immer notwendiger Austausch des Multi-Lumen-Schlauchs vereinfacht und ist somit in kürzerer Zeit ausführbar. Allerdings kann das üblicherweise aus starrerem Material als der Multi-Lumen-Schlauch hergestellte Gehäuse der Ventilbaugruppe der sie durchsetzenden Leitungsanordnung eine bessere Zugentlastung bieten als der in der Regel weichelastische Multi-Lumen-Schlauch. Eine Durchsetzung des Multi-Lumen-Schlauchs durch die Leitungsanordnung kann an nur einem Längsendbereich oder kann an beiden Längsendbereichen vorgesehen sein. Bei einer Durchsetzung des Beatmungsschlauchs am distalen Längsende kann die Leitungsanordnung unter Umgehung von zwischen dem Beatmungsgerät und dem Beatmungsschlauch angeordneten Komponenten, wie etwa einem Exspirationsventil, unmittelbar mit dem Beatmungsgerät gekoppelt sein.

Gemäß einer wegen ihres geringen Strömungswiderstands bevorzugten Ausführungsform des Exspirationsventils ist im betriebsbereiten Zustand der Beatmungsschlauch mit einer Anschlussformation des Exspirationsventils, insbesondere eines Grundkörpers des Exspirationsventils, atemgasleitend verbunden. Die im Beatmungsschlauch geführte Leitungsanordnung ist zur Vermeidung unnötiger Umlenkung in der Leitungsanordnung bevorzugt durch die Anschlussformation hindurch in das Exspirationsventil, insbesondere in den Grundkörper des Exspirationsventils, hinein geführt. Im Exspirationsventil ausgebildete Strömungskanäle für einmal exspiratorisches Atemgas und einmal inspiratorisches Atemgas sind vorzugsweise längs ihres Verlaufs vom Beatmungsschlauch bzw. von der Anschlussformation zur Verbindung des Exspirationsventils mit dem Beatmungsschlauch weg in das Gehäuse des Exspirationsventils hinein abgewinkelt, sodass die Leitungsanordnung möglichst ohne Umlenkung unter Fortsetzung ihres Verlaufs im Bereich der Anschlussformation des Exspirationsventils zur Verbindung mit dem Beatmungsschlauch in das Gehäuse des Exspirationsventils hinein geführt werden kann, ohne auf den Ventilkörper des Exspirationsventils zu treffen und ohne im inspiratorischen Strömungskanal im Gehäuse des Exspirationsventils in ein Beatmungsgerät geführt zu werden. Damit der inspiratorische und der exspiratorische Strömungskanal einander im Gehäuse, vorzugsweise im Grundkörper, des Exspirationsventils möglichst nicht stören, sind die beiden Strömungskanäle längs ihres Verlaufs von der Anschlussformation weg in das Exspirationsventilgehäuse hinein in unterschiedliche, vorzugweise in entgegengesetzte Richtungen, abgewinkelt.

Die Anmelderin behält sich vor, selbständigen Schutz für ein Exspirationsventil zu beanspruchen, wie es in der vorliegenden Anmeldung beschrieben ist.

Um die Leitungsanordnung am distalen Längsende also im Bereich des Exspirationsventils für eine Verbindung mit dem Beatmungsgerät oder eine Erfassungsvorrichtung zugänglich zu machen ist bevorzugt am Gehäuse des Exspirationsventils eine Anschlussstutzenanordnung vorgesehen, von welcher eine innere Anschlussstutzenanordnung mit der Leitungsanordnung verbindbar sein kann und von welcher eine äußere Anschlussstutzenanordnung zur Verbindung mit dem Beatmungsgerät oder der Erfassungsvorrichtung ausgebildet sein kann. Die Anschlussstutzenanordnung ist zur Vermeidung von unnötigen Umlenkungen und damit Strömungswiderständen der Leitungsanordnung bevorzugt als geradlinige rohrförmige Anschlussstutzenanordnung mit je einem rohrförmigen Anschlussstutzen für jede Leitung der Leitungsanordnung ausgebildet, wobei die Anschlussstutzenanordnung das Gehäuse des Exspirationsventils durchsetzt. Bevorzugt durchsetzt die Anschlussstutzenanordnung das Gehäuse des Exspirationsventils auf der von der Anschlussformation des Exspirationsventils zur Verbindung mit dem Beatmungsschlauch abgewandten Seite. Durch Spritzgießen kann wenigstens ein Gehäusebauteil des Exspirationsventils einstückig mit der Anschlussstutzenanordnung ausgebildet sein.

Die Rohrachse der rohrförmigen Anschlussstutzen am Exspirationsventil verlaufen zur Vermeidung von unerwünschten Umlenkungen der Leitungsanordnung vorzugsweise parallel zum Verlauf des inspiratorischen oder/und des exspiratorischen Strömungspfads im Bereich der Anschlussformation des Exspirationsventils zur Verbindung mit dem Beatmungsschlauch.

Eine unerwünschte Vergrößerung des vom Exspirationsventil eingenommenen Bauraums durch die auf der von der genannten Anschlussformation abgewandten Seite angeordnete äußere Anschlussformation kann dadurch vermieden werden, dass die äußere Anschlussstutzenanordnung in einem exspiratorisch stromabwärts des Exspirationsventilkörpers gelegenen Auslassbereich angeordnet ist, in welchem vom Exspirationsventil exspiratorisches Atemgas an die Außenumgebung des Exspirationsventils abgegeben wird. Dann kann die äußere Anschlussstutzenanordnung in einer Auslassausnehmung des Exspirationsventilgehäuses aufgenommen sein.

Wegen seiner besonderen Bedeutung betrifft die vorliegende Erfindung auch einen Beatmungsschlauch mit wenigstens zwei, vorzugsweise genau zwei, in einem gemeinsamen Schlauchkörper ausgebildeten Lumina, und mit einer Leitungsanordnung, welche eine ein Lumen begrenzende Schlauchwand in deren Dickenrichtung durchsetzt und über einen Längsabschnitt des Schlauchkörpers in wenigstens einem der Lumina verläuft. Der Beatmungsschlauch kann als Multi-Lumen-Schlauch entsprechend dem in dieser Anmeldung beschriebenen Multi-Lumen-Schlauch weitergebildet sein. Insbesondere sind die Lumina des Beatmungsschlauchs bevorzugt durch ein sowohl in Beatmungsschlauch-Längsrichtung als auch orthogonal dazu verlaufendes Septum voneinander getrennt, welches eine gemeinsame Trennwand für wenigstens zwei aneinander angrenzende Lumina bildet. Außerhalb des Septums sind bei dieser bevorzugten Ausführungsform zwei aneinander angrenzende Lumina durch jeweils eine Schlauchwand umgrenzt, die keine gemeinsame Trennwand zwischen den beiden Lumina ist. Nur das Septum wird dann beiderseits von Atemgas benetzt, die übrigen Schlauchwandabschnitte dagegen nicht.

Die Leitungsanordnung des Beatmungsschlauchs kann entsprechend den übrigen Darlegungen zur Leitungsanordnung in dieser Anmeldung weitergebildet sein.

Bevorzugt ist die Durchsetzung des Beatmungsschlauchs in einem oder in beiden Längsendabschnitten des Beatmungsschlauchs ausgebildet, also etwa auf den letzten 25 %, bevorzugt 15 %, besonders bevorzugt 10 %, der Gesamtlänge des Beatmungsschlauchs. Ist nur eine Durchsetzung ausgebildet, verläuft die Leitungsanordnung bevorzugt von der Durchsetzung bis zu dem von der Durchsetzung weiter entfernt gelegenen Längsende des Beatmungsschlauchs in einem Lumen oder in mehreren, vorzugsweise in beiden Lumina des Beatmungsschlauchs. Sind dagegen zwei Durchsetzungen längs der Beatmungsschlauch-Längsrichtung mit Abstand voneinander ausgebildet, verläuft bevorzugt die Leitungsanordnung zwischen den Durchsetzungen in einem Lumen oder in mehreren, vorzugsweise in beiden Lumina des Beatmungsschlauchs.

Die vorliegende Anmeldung betrifft außerdem eine Baugruppe, umfassend einen wie oben beschrieben ausgestalteten Multi-Lumen-, vorzugsweise Bi-Lumen-Beatmungsschlauch sowie eine Atemgas-Ventilbaugruppe oder/und einen Druckdifferenz-Durchflusssensor. Die Atemgas-Ventilbaugruppe oder/und der Druckdifferenz-Durchflusssensor ist bzw. sind an einem Längsende des Multi-Lumen-Beatmungsschlauchs derart angeschlossen, dass Atemgas in zwei entgegengesetzte Richtungen durch die Baugruppe hindurchleitbar ist. Der Druckdifferenz-Durchflusssensor kann unmittelbar oder, was aus den oben genannten Gründen bevorzugt ist, unter Zwischenanordnung der Ventilbaugruppe an den Beatmungsschlauch angeschlossen sein. Die Leitungsbaugruppe ist bevorzugt an den Druckdifferenz-Durchflusssensor zur Informationsübertragung angeschlossen, wobei die von der Leitungsbaugruppe zu übertragende Information elektrische Signale oder pneumatische Information, etwa in Gestalt eines übertragenen Atemgasdrucks, sein können.

Bevorzugt befindet sich der Multi-Lumen-Schlauch zwischen der Atemgasquelle und der Atemgas-Ventilbaugruppe, wobei die Ventilbaugruppe auf der proximalen Seite und die Atemgasquelle auf der distalen Seite des Multi-Lumen-Schlauchs angeordnet sind. Zur besseren Steuerung der Exspiration, insbesondere zur Bereitstellung eines definierten PEEP ist es vorteilhaft, wenn der Multi-Lumen-Schlauch die proximale Atemgas-Ventilbaugruppe mit einem distalen Exspirationsventil atemgasleitend verbindet. Der Multi-Lumen-Schlauch befindet sich dann wiederum zwischen Ventilbaugruppe und Exspirationsventil. Zur Unterscheidung vom Exspirationsventil, das in der Regel ein Membranventil mit einer von einem Ventilsitz abhebbaren Ventilmembran ist, ist das dem exspiratorischen Strömungspfad zugeordnete Ventil der Ventilanordnung als "exspiratorisches Ventil" bezeichnet.

Zum sicheren Sperren des exspiratorischen Strömungspfads zusätzlich zum exspiratorischen Ventil kann die Ventilmembran des als Membranventil ausgebildeten Exspirationsventils auf der dem Multi-Lumen-Schlauch zugewandten Seite mit exspiratorischem Atemgas beaufschlagbar sein und kann die Ventilmembran auf der dem Multi-Lumen-Schlauch abgewandten Seite mit inspiratorischem Atemgas beaufschlagbar sein. Dann kann durch den bevorzugt nur während der Inspirationsphase erhöhten Druck des inspiratorischen Atemgases das Exspirationsventil während der Inspirationsphase geschlossen gehalten werden.

Das Exspirationsventilgehäuse kann hierzu mehrteilig ausgeführt sein, mit einem Grundkörper und einem am Grundkörper festlegbaren Deckel. Vorzugsweise umfasst der Grundkörper je einen exspiratorischen Strömungskanal und einen inspiratorischen Strömungskanal sowie einen Ventilsitz für das Membranventil. Der Deckel fixiert vorzugsweise die Ventilmembran am Grundkörper des Exspirationsventils oder trägt wenigstens zu deren Fixierung am Grundkörper bei. Zwischen dem Deckel und der Ventilmembran, auf der vom Ventilsitz abgewandten Seite der Ventilmembran, kann ein Steuerraum gebildet sein, welcher fluidmechanisch mit dem inspiratorischen Strömungspfad kommuniziert, so dass im Steuerraum während einer Inspriationsphase ein erhöhter Gasdruck herrscht, welcher die Ventilmembran gegen den Ventilsitz drückt und so die Verschlussdichtigkeit des Exspirationsventils während einer Inspirationsphase erhöht.

Die vorliegende Erfindung wird nachfolgend anhand der beiliegenden Zeichnungen näher erläutert werden. Es stellt dar:
- Fig.1: eine grobschematische Darstellung einer erfindungsgemäßen Beatmungsvorrichtung,
- Fig. 2: eine grobschematische perspektivische Darstellung eines Abschnitts der Beatmungsleitungsanordnung von Figur 1, umfassend einen proximalen Endbereich des Beatmungsschlauchs, die an dessen proximalen Endbereich angeschlossene erfindungsgemäße Atemgas-Ventilbaugruppe und einen proximal an diese angeschlossenen Differenzdruck-Durchflusssensor,
- Fig. 3: eine grobschematische Längsschnittansicht des proximalen Endbereichs des Beatmungsschlauchs und der daran angeschlossenen Ventilbaugruppe von Figur 2,
- Fig. 4: eine grobschematische perspektivische Ansicht des Exspirationsventils von Figur 1 sowie des distalen Endbereichs des Beatmungsschlauchs,
- Fig. 5: eine grobschematische Längsschnittansicht des proximalen Endbereichs des Beatmungsschlauchs und des Exspirationsventils,
- Fig. 6: eine grobschematische Explosions-Aufrissansicht einer zweiten Ausführungsform eines Exspirationsventils,
- Fig. 7: eine grobschematische Aufrissansicht des Grundkörpers des Exspirationsventils von Figur 6 von hinten, also längs der Blickrichtung gemäß Pfeil VII von Figur 6,
- Fig. 8: eine grobschematische Draufsicht auf den Grundkörper des Exspirationsventils von Figur 6 längs der Blickrichtung gemäß Pfeil VIII von Figur 6,
- Fig. 9: eine grobschematische Schnittansicht durch den Grundkörper des Exspirationsventils von Figur 6 längs der Schnittebene IX-IX von Figur 7,
- Fig. 10: eine grobschematische Schnittansicht längs der Schnittebene X-X von Figur 8, und
- Fig. 11: eine grobschematische Explosionsansicht einer zweiten Ausführungsform einer erfindungsgemäßen Atemgas-Ventilbaugruppe.

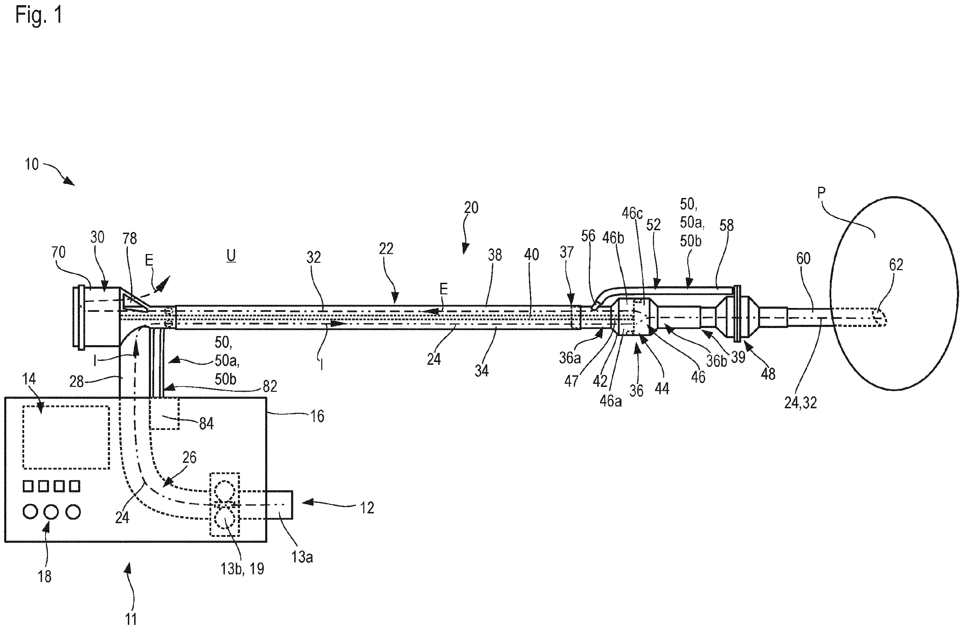

In Figur 1 ist eine erfindungsgemäße Ausführungsform einer Beatmungsvorrichtung allgemein mit 10 bezeichnet. Die Beatmungsvorrichtung 10 umfasst ein tragbares Beatmungsgerät 11 mit einer Atemgasquelle 12 in Gestalt eines Umgebungsluft-Ansaugstutzens 13a und eines Gebläses 13b sowie mit einer Steuervorrichtung 14 zur Einstellung von Betriebsparametern der Atemgasquelle 12. Die Atemgasquelle 12 und die Steuervorrichtung 14 sind im selben Gehäuse 16 des Beatmungsgeräts 11 aufgenommen.

Die Steuervorrichtung 14 der Beatmungsvorrichtung 10 weist eine Eingabe/Ausgabeeinrichtung 18 auf, welche zahlreiche Schalter, wie Tastschalter und Drehschalter umfasst, um erforderlichenfalls Daten und Steuerbefehle in die Steuervorrichtung 14 eingeben zu können. Das Gebläse 13b der Atemgasquelle 12 kann in seiner Förderleistung durch die Steuervorrichtung 14 verändert werden, um die Menge an inspiratorischem Atemgas, das von der Atemgasquelle 12 pro Zeiteinheit gefördert wird, zu verändern. Das Gebläse 13b ist daher im vorliegenden Ausführungsbeispiel auch eine Druckveränderungsvorrichtung 19 der Beatmungsvorrichtung 10.

An die Atemgasquelle 12 ist eine Beatmungsleitungsanordnung 20 angeschlossen, welche im vorliegenden Beispiel mehrere Längsabschnitte, darunter einen flexiblen Bi-Lumen-Beatmungsschlauch 22, umfasst.

Ein in Inspirationsrichtung I längs eines inspiratorischen Strömungspfads 24 erster Abschnitt 26 der Beatmungsleitungsanordnung 20 verläuft im Inneren des Gehäuses 16 vom Gebläse 13b zu einem im vorliegenden Beispiel, aber nicht grundsätzlich, außerhalb des Gehäuses 16 gelegenen Verbindungskanal 28, welcher die gehäuseinterne inspiratorische Atemgasleitung 26 mit einem Exspirationsventil 30 verbindet. Wie weiter unten noch erläutert werden wird, wird im Exspirationsventil 30 inspiratorisches Atemgas dazu verwendet, während einer Inspirationsphase einen Membran-Ventilkörper 76 auf seinen Ventilsitz 77 zu drücken und so den exspiratorischen Strömungspfad 32 im Bereich des Exspirationsventils 30 gegen Durchströmung in Exspirationsrichtung E zu sperren. Das inspiratorische Atemgas strömt im Exspirationsventil 30 an exspiratorisches Atemgas führenden Leitungen vorbei.

In Inspirationsrichtung I längs des inspiratorischen Strömungspfads 24 folgt auf das Exspirationsventil 30 der Bi-Lumen-Beatmungsschlauch 22, in dessen inspiratorischem Lumen 34 ausschließlich inspiratorisches Atemgas vom Exspirationsventil 30 zu einer Atemgas-Ventilbaugruppe 36 strömen kann.

Ein dem inspiratorischen Lumen 34 benachbartes und zu diesem paralleles exspiratorisches Lumen 38, durch welches ein Abschnitt des exspiratorischen Strömungspfads 32 verläuft, ist vom inspiratorischen Lumen 34 durch ein in Figur 1 nur strichliniert gezeichnetes Septum 40 getrennt. Im exspiratorischen Lumen 38 strömt im Betrieb nur exspiratorisches Atemgas.

Die Ventilbaugruppe 36 weist ein Gehäuse 42 auf, in welchem eine weiter unten noch detaillierter beschriebene Ventilanordnung 44 aufgenommen ist. Das Gehäuse 42 wird von einer Kanalanordnung 46 durchsetzt, wobei die Kanalanordnung 46 auf der baulich distalen Seite 36a der Ventilbaugruppe 36 bezüglich der Ventilanordnung 44 als Bi-Lumen-Kanalanordnung mit einem inspiratorischen Kanallumen 46a zur Führung von nur inspiratorischem Atemgas und mit einem davon durch ein Kanalseptum 47 getrennten exspiratorischen Kanallumen 46b zur Führung von nur exspiratorischem Atemgas ausgebildet ist. Das inspiratorische Kanallumen 46a ist baulich eine Fortsetzung des inspiratorischen Lumens 34. Ebenso ist das exspiratorische Kanallumen 46b baulich eine Fortsetzung des exspiratorischen Lumens 38.

Auf der baulich proximalen Seite 36b der Ventilbaugruppe 36 bezüglich der Ventilanordnung 44 ist die Kanalanordnung 46 als gemeinsamer Strömungsführungsabschnitt bzw. Kanalabschnitt 46c sowohl für inspiratorisches als auch für exspiratorisches Atemgas ausgebildet.

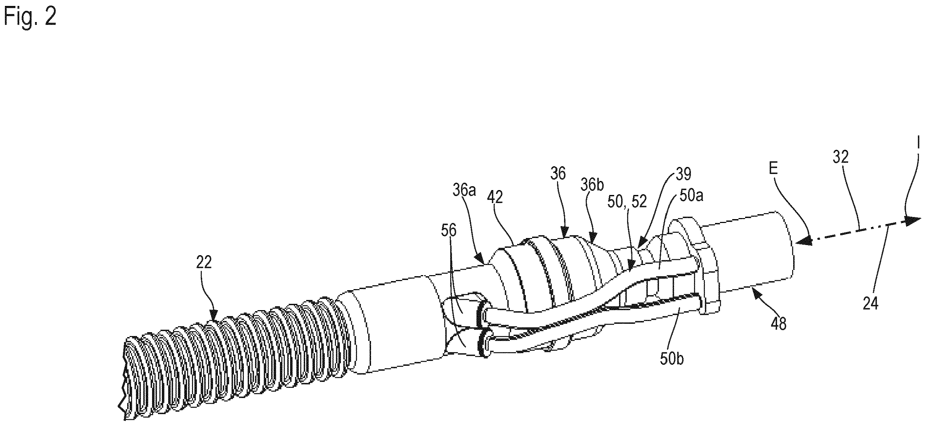

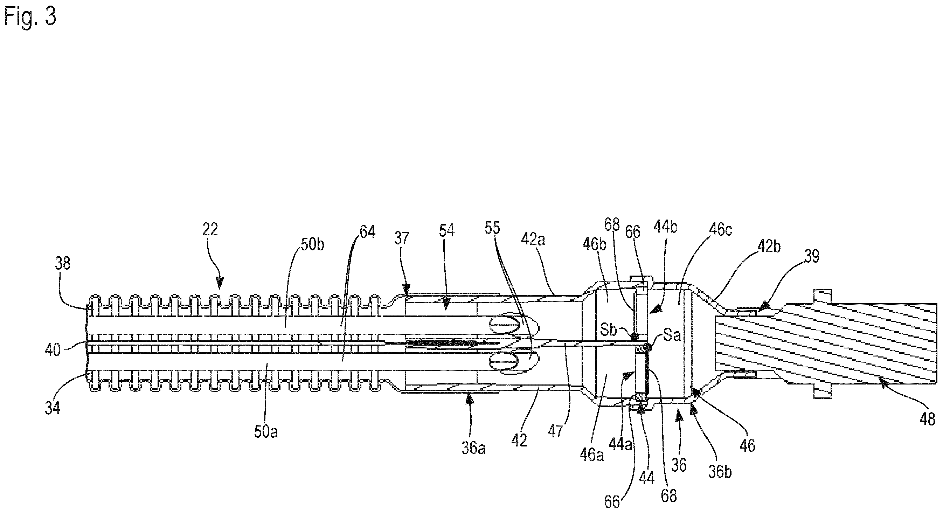

Das Septum 40 des Bi-Lumen-Schlauchs 22 und das Kanalseptum 47 sind in Figur 1 lediglich grobschematisch dargestellt, um ihre Präsenz im Inneren der beteiligten Bauteile darzustellen. In den nachfolgenden Figuren 2 und 3 sind die Septa 40 und 47 bezüglich der Längsachse der in Figur 1 gezeigten Beatmungsleitungsanordnung 20 um 90° gedreht, sodass gemäß den Darstellungen der Figuren 2 und 3 das inspiratorische Lumen 34 und das exspiratorische Lumen 38 sowie das inspiratorische Kanallumen 46a und das exspiratorische Kanallumen 46b in Betrachtungsrichtung der Figur 1 hintereinander anstelle der in Figur 1 gezeigten Übereinander-Anordnung liegen. Die Septa 40 und 47 gemäß den Figuren 2 und 3 sind bei Übertragung der Darstellung auf die Figur 1, nicht wie in Figur 1 gezeigt orthogonal zur Zeichenebene von Figur 1, sondern parallel dazu ausgebildet. Beide Anordnungen der Septa 40 und 47 und der durch sie getrennten Lumina 34 und 38 sowie 46a und 46b sind in der Realität möglich.

Im inspiratorischen Kanallumen 46a als einem gesonderten Strömungsführungsabschnitt verläuft nur der inspiratorische Strömungspfad 24. Im exspiratorischen Kanallumen 46b als einem weiteren gesonderten Strömungsführungsabschnitt verläuft nur der exspiratorische Strömungspfad 32. Im gemeinsamen Kanalabschnitt 46c verlaufen sowohl der inspiratorische Strömungspfad 24 als auch der exspiratorische Strömungspfad 32.

An die proximale, kombiniert inspiratorisch-exspiratorische bauliche Seite 36b der Ventilbaugruppe 36 schließt im dargestellten Beispiel, aber nicht notwendigerweise grundsätzlich, ein Durchflusssensor 48 an. Im dargestellten Beispiel ist der Durchflusssensor 48 durch einen bevorzugten Differenzdruck-Durchflusssensor 48 gebildet, welcher den inspiratorischen und den exspiratorischen Fluss an Atemgas zum Patienten P hin und vom Patienten P weg erfasst. Eine Leitungsanordnung 50 überträgt die beiderseits eines Strömungshindernisses im Durchflusssensor 48 herrschenden Gasdrücke an die Steuervorrichtung 14, bzw. an deren Drucksensor 84, wobei die Steuervorrichtung 14 aus den übertragenen Gasdrücken und insbesondere aus der Differenz der Gasdrücke die Menge an pro Zeiteinheit strömendem inspiratorischem und exspiratorischem Atemgas errechnet. Die Leitungsanordnung 50 umfasst zwei Leitungen 50a und 50b, für jeden zu übertragenden Gasdruck je eine.

Zu erkennen in Figur 1 ist nur ein äußerer Teil 52 der Leitungsanordnung 50, da die Leitungsanordnung 50 das Gehäuse 42 der Ventilbaugruppe 36 im Bereich zwischen dem distalen Ende 37 der Ventilbaugruppe 36 und der Ventilanordnung 44 durchsetzt. Ab dem Ort der Durchsetzung verläuft ein innerer Teil 54 (siehe Figur 3) der Leitungsanordnung 50 in Richtung von der Ventilanordnung 44 weg im Inneren des Gehäuses 42 und im Inneren des Bi-Lumen-Schlauchs 22. Das distale Ende 37 der Ventilbaugruppe 36 ist distaler inspiratorischer Einlassendbereich und ist distaler exspiratorischer Auslassendbereich. Das proximale Ende der Ventilbaugruppe 36, welches proximaler inspiratorischer Auslassendbereich und proximaler exspiratorischer Einlassendbereich ist, ist mit Bezugszeichen 39 bezeichnet.

Am Ort der Durchsetzung sind am Gehäuse 42 äußere Anschlussstutzen 56 ausgebildet, an welche zur Bildung des äußeren Teils 52 der Leitungsanordnung 50 beitragende flexible Schläuche 58 durch Aufstecken angeschlossen sind. Andernends sind die flexiblen Schläuche 58 der Leitungsanordnung 50 in an sich bekannter Weise am Durchflusssensor 48 angeschlossen.

Alternativ kann die Leitungsanordnung 50 unterbrechungsfrei durch eine das Gehäuse 42 durchsetzende Öffnungsanordnung hindurchgeführt sein. Die Leitungsanordnung 50 ist dann bevorzugt gegen die durchsetzte Öffnungsanordnung gasdicht abgedichtet, etwa durch eine um die Leitungsanordnung 50 umlaufende Dichtung oder durch zwei um ihre Einzelleitungen 50a und 50b umlaufende Dichtungen. Diese wenigstens eine Dichtung kann ortsfest an der durchsetzten Öffnungsanordnung angeordnet sein.

Das Gehäuse 42 ist vorliegend wenigstens zweiteilig ausgebildet und weist ein distales Gehäusebauteil 42a und ein proximales Gehäusebauteil 42b auf (siehe Figur 3). Die beiden Gehäusebauteile 42a und 42b können durch eine Steckverbindung miteinander zum Gehäuse 42 verbunden sein. Zusätzlich zu der Steckverbindung, bei welcher beispielsweise ein proximaler Endabschnitt des distalen Gehäusebauteils 42a in einen distalen Endabschnitt des proximalen Gehäusebauteils 42b eingesteckt sein kann, können die beiden Gehäusebauteile 42a und 42b in dem so gebildeten Überlappungsbereich stoffschlüssig verbunden sein, etwa durch Kleben oder Schweißen, insbesondere Ultraschallschweißen.

Auf den Durchflusssensor 48 folgt in Richtung zum Patienten P hin ein Endotrachealtubus 60 als Beatmungsschnittstelle zum Patienten P. Eine proximale Öffnung 62 des Endotrachealtubus 60 ist sowohl Atemgas-Auslassöffnung, durch welche inspiratorisches Atemgas durch den Endotrachealtubus 60 in den Patienten P eingeleitet wird, als auch Atemgas-Einlassöffnung, durch welche exspiratorisches Atemgas aus dem Patienten P zurück in den Endotrachealtubus 60 geleitet wird. Folglich verlaufen durch den Endotrachealtubus 60 sowohl der inspiratorische Strömungspfad 24 als auch der exspiratorische Strömungspfad 32.

Der Endotrachealtubus 60 ist in Figur 1 lediglich grobschematisch dargestellt. Zwischen dem Durchflusssensor 48 und dem Endotrachealtubus 60 kann wenigstens ein weiterer Schlauchabschnitt eines Beatmungsschlauchs angeordnet sein.

In Figur 2 ist ein Abschnitt der Beatmungsleitungsanordnung 20 von Figur 1 perspektivisch von schräg oben gezeigt. Der Abschnitt umfasst einen proximalen Endabschnitt des Bi-Lumen-Schlauchs 22, die Ventilbaugruppe 36 und den Durchflusssensor 48. Zu erkennen sind die nebeneinander verlaufenden flexiblen Schläuche 58 des äußeren Teils 52 der beiden Leitungen 50a und 50b der Leitungsanordnung 50 vom Durchflusssensor 48 zu den äußeren Anschlussstutzen 56 in der baulich distalen Seite 36a der Ventilbaugruppe 36.

In Figur 3 ist ein Längsschnitt durch den in Figur 2 gezeigten Abschnitt der Beatmungsleitungsanordnung 20 dargestellt. Zu erkennen sind auf der distalen baulichen Seite 36a innere Anschlussstutzen 55, an welche innere flexible Schläuche 64 als innerer Teil 54 der Leitungsanordnung 50 angeschlossen sind. Im dargestellten Beispiel verläuft in jedem der Lumina 34 und 38 je eine Leitungsanordnung 50a bzw. 50b, was bevorzugt ist, da bei identischen Schläuchen 64 und spiegelbildlich bezüglich des Septums 40 ausgebildeten Lumina 34 und 38 von jedem Lumen 34 und 38 nach Verlegung des inneren Teils 54 der Leitungsanordnung ein etwaiges gleichgroßes Restvolumen zur Durchleitung von Atemgas verbleibt.

Da der Durchflusssensor 48 an sich bekannt ist, ist dieser in Figur 3 nicht im Detail dargestellt sondern lediglich als Baugruppe symbolisiert.

Das Septum 40 des Bi-Lumen-Schlauchs 22 ist mit dem Kanalseptum 47 der Ventilbaugruppe 36 formschlüssig zur Bildung einer durchgängigen Lumenwand verbindbar. Hierzu kann eines der beiden Septa, vorzugsweise das aus steiferem Material ausgebildete Kanalseptum 47, eine Ausnehmung aufweisen, in welche ein Vorsprung des jeweils anderen Septums, im dargestellten Beispiel des Septums 40, einragt.

In Figur 3 ist außerdem die Ventilanordnung 44 im Detail dargestellt. Sie umfasst ein inspiratorisches Ventil 44a und ein exspiratorisches Ventil 44b, welche konstruktiv im Wesentlichen identisch ausgebildet und lediglich unterschiedlich orientiert angeordnet sind.

Sowohl das inspiratorische Ventil 44a als auch das exspiratorische Ventil 44b weisen jeweils einen Ventilsitz 66 auf, etwa gebildet durch einen geschlossen umlaufenden Ring aus weichelastischen Polymer, beispielsweise aus Silikongummi, an welchem in der Sperrstellung des jeweiligen Ventils ein Ventilkörper 68 in Gestalt einer Platte anliegt. Der Ventilkörper 68 ist aus steiferem Material gebildet als der Ventilsitz 66.

In Figur 3 sind beide Ventile 44a und 44b in ihrer Sperrstellung dargestellt, in welcher sie eine Durchströmung der Ventilanordnung 44 durch Atemgas sperren. Beide Ventilkörper 68 sind im dargestellten Ausführungsbeispiel in die dargestellte Sperrstellung vorgespannt. Beide Ventilkörper 68 sind relativ zum jeweils zugeordneten Ventilsitz 66 um eine zur Zeichenebene der Figur 3 orthogonale Schwenkachse Sa bzw. Sb schwenkbar angeordnet, wobei die beiden Ventilkörper 68 ausgehend von der dargestellten Sperrstellung relativ zu ihrem jeweils zugeordneten Ventilsitz 66 funktionsbedingt in entgegengesetzte Richtungen schwenken, um das durch sie zunächst gesperrte Lumen: Lumen 34 bzw. Kanallumen 46a sowie Lumen 38 bzw. Kanallumen 46b, für Atemgas durchströmbar zu machen. Aus diesem Grunde sind die beiden Ventilkörper 68 auf unterschiedlichen Seiten ihrer jeweiligen Ventilsitze 66 angeordnet. Konstruktiv bedingt können die beiden Ventilkörper 68 ausgehend von ihrer Sperrstellung jeweils nur in genau einer Richtung von ihrem jeweils zugeordneten Ventilsitz 66 abheben. Die Abheberichtungen der beiden Ventilkörper 68 sind einander entgegengesetzt.

Die Vorspannung der Ventilkörper 68 in ihre Sperrstellung ist so eingestellt, dass die Vorspannung des Ventilkörpers 68 des inspiratorischen Ventils 44a durch den vom Gebläse 13b erzeugten Atemgasdruck überwunden werden kann, und dass die Vorspannung des Ventilkörpers 68 des exspiratorischen Ventils 44b durch den Atemgasdruck der Patientenlunge nach Abschluss einer Inspirationsphase überwunden werden kann. Aufgrund der gewählten Konstruktion der Ventile 44a und 44b als Rückschlag-Klappenventile und weiter aufgrund ihrer Anordnung, wirkt ein erhöhter Druck von exspiratorischem Atemgas Schließkraft erhöhend auf das inspiratorische Ventil 44a und wirkt ein erhöhter Druck von inspiratorischem Atemgas im exspiratorischen Kanallumen 46b Schließkraft erhöhend auf das exspiratorische Ventil 44b.

In der Beatmungsvorrichtung 10 von Figur 1 ist das inspiratorische Ventil 44a das einzige Ventil der Beatmungsvorrichtung 10, welches eine den inspiratorischen Strömungspfad 24 unterbrechende Wirkung aufweist. Ein weiteres Inspirationsventil ist in der Beatmungsvorrichtung 10 nicht vorgesehen.

Im Gegensatz dazu ist zusätzlich zum exspiratorischen Ventil 44b das bereits oben genannte Exspirationsventil 30 in der Beatmungsvorrichtung 10 vorgesehen, und zwar angeschlossen am proximalen Längsende des Bi-Lumen-Schlauchs 22.

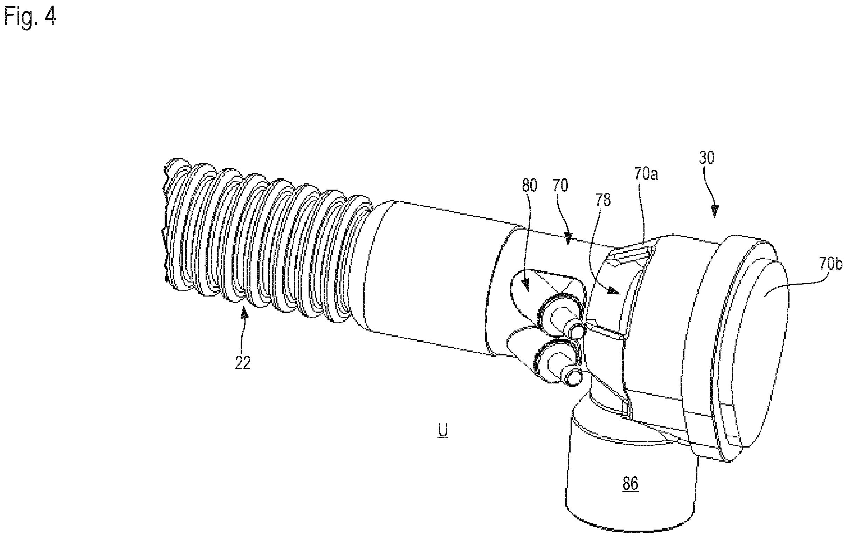

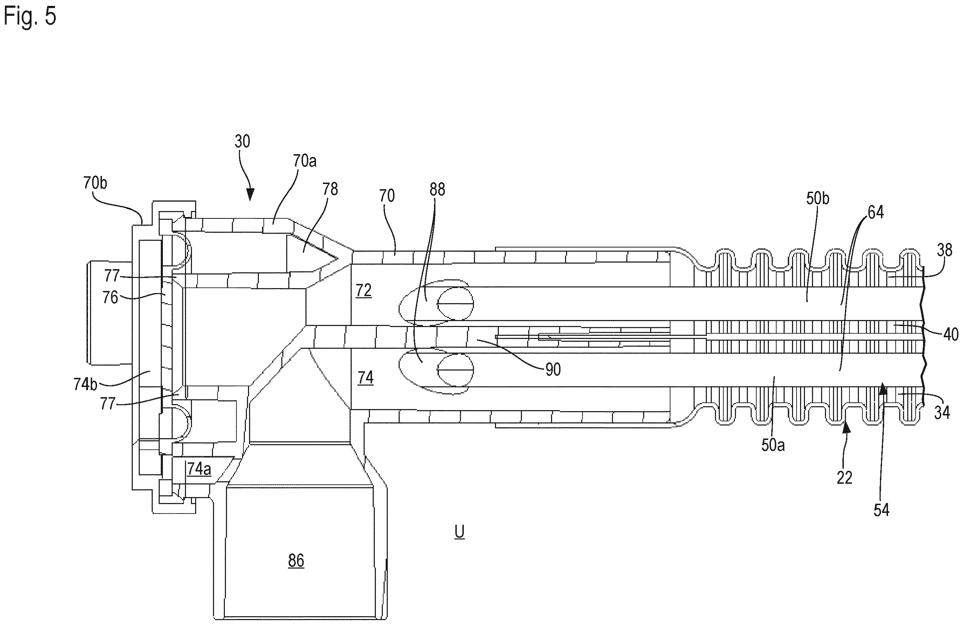

Das Exspirationsventil 30 wird nachfolgend in Zusammenhang mit den Figuren 4 und 5 näher erläutert werden.

Das Exspirationsventil 30 weist ein Gehäuse 70 auf, in welchem ein Exspirationskanal 72 und ein Inspirationskanal 74 ausgebildet sind. Der Exspirationskanal 72 ist in an sich bekannter Weise durch einen in die in Figur 5 dargestellte Schließstellung vorgespannte Membran-Ventilkörper 76 verschlossen und kann durch Erhöhung des Drucks des exspiratorischen Atemgases im exspiratorischen Lumen 38 bezüglich des Umgebungsdrucks geöffnet werden. Im Gehäuse 70 sind das Gehäuse 70 in Dickenrichtung durchsetzende Fenster 78 ausgebildet, durch welche exspiratorisches Atemgas bei vom Ventilsitz 77 abgehobenen Membran-Ventilkörper 76 aus dem Gehäuse 70 in die Umgebung U entweichen kann.

Am Gehäuse 70 des Exspirationsventils 30 sind äußere Anschlussstutzen 80 ausgebildet, an welche flexible Schläuche 82 zur Verbindung mit einem Drucksensor 84 der Steuervorrichtung 14 angeschlossen sind, um die vom Durchflusssensor 48 durch die Leitungsanordnung 50 übertragenen Drücke quantitativ zu ermitteln und hieraus die pro Zeiteinheit geströmte Menge an Atemgas zu bestimmen. Für die äußeren Anschlussstutzen 80 gilt das oben zu den äußeren Anschlussstutzen 56 Gesagte entsprechend.

Das Gehäuse des Exspirationsventils 30 weist weiter eine Anschlussformation 86 als Teil des Verbindungskanals 28 zum Anschluss einer Atemgas-Verbindungsleitung zwischen dem Beatmungsgerät 11 und dem Exspirationsventil 30 auf.

Vom Inspirationskanal 74 im Gehäuse 70 zweigt ein Steuerkanal 74a ab, welcher zum Membran-Ventilkörper 76 führt. Durch den Steuerkanal 74a ist die vom exspiratorischen Atemgas im Exspirationskanal 72 abgewandte Seite des Membran-Ventilkörpers 76 mit inspiratorischem Atemgas beaufschlagbar, sodass der Druck des inspiratorischen Atemgases als Steuerdruck für das Exspirationsventil 30 verwendet werden kann. Wie bereits oben beschrieben wurde, liefert das Gebläse 13b nur während einer Inspirationsphase einen erhöhten Druck des inspiratorischen Atemgases und ist während der Exspirationsphase abgeschaltet oder hinsichtlich seiner Leistung stark gedrosselt.

In Figur 5 sind innere Anschlussstutzen 88 des Gehäuses 70 zu erkennen, an welchen die flexiblen Schläuche 64 des inneren Teils 54 der Leitungsanordnung 50 angeschlossen sind. Für die inneren Anschlussstutzen 88 gilt das zu den inneren Anschlussstutzen 55 Gesagte entsprechend, und umgekehrt. Die inneren Anschlussstutzen 55 und 88 sind bevorzugt jeweils einstückig mit ihrem Gehäuse ausgebildet, beispielsweise durch Spritzgießen.

Das Gehäuse 70 weist außerdem ein Gehäuseseptum 90 auf, an welches das Septum 40 des Bi-Lumen-Schlauchs 22 spaltfrei anschließt. Das Gehäuseseptum 90 trennt im Gehäuse 70 den Exspirationskanal 72 vom Inspirationskanal 74.

Das Gehäuse 70 des Exspirationsventils 30 ist vorliegend zweiteilig ausgebildet mit einem kanalführenden Grundkörper 70a, an welchem auch der Ventilsitz 77 vorzugsweise einstückig ausgebildet ist, und mit einem Deckel 70b. Zwischen dem Deckel 70b und dem kanalführenden Grundkörper 70a ist der Membran-Ventilkörper 76 gehalten. Auf der vom Ventilsitz 77 und vom Exspirationskanal 72 abgewandten Seite des Membran-Ventilkörpers 76 befindet sich ein Steuerraum 74b, welcher durch den Steuerkanal 74a mit inspiratorischem Atemgas beaufschlagbar ist, sodass während einer Inspirationsphase im Steuerraum 74b ein erhöhter Gasdruck herrscht, durch welchen der Membran-Ventilkörper 76 gegen den Ventilsitz 77 gedrückt wird. Der erhöhte Gasdruck im Steuerraum 74b sorgt somit während einer Inspirationsphase, während welcher sich der Exspirationskanal 72 im Wesentlichen auf Umgebungsdruckniveau befindet, für eine erhöhte Dichtigkeit der Anlage des Membran-Ventilkörper 76 am Ventilsitz 77.

Durch das exspiratorische Ventil 44b kann die Luftsäule im exspiratorischen Lumen 38 während einer Inspirationsphase strömungsmechanisch vom übrigen Atemgas getrennt werden, sodass vom Membran-Ventilkörper 76 etwaig in der Luftsäule angeregte Schwingungen den Patienten P nicht erreichen und so den Inspirationsvorgang nicht stören.

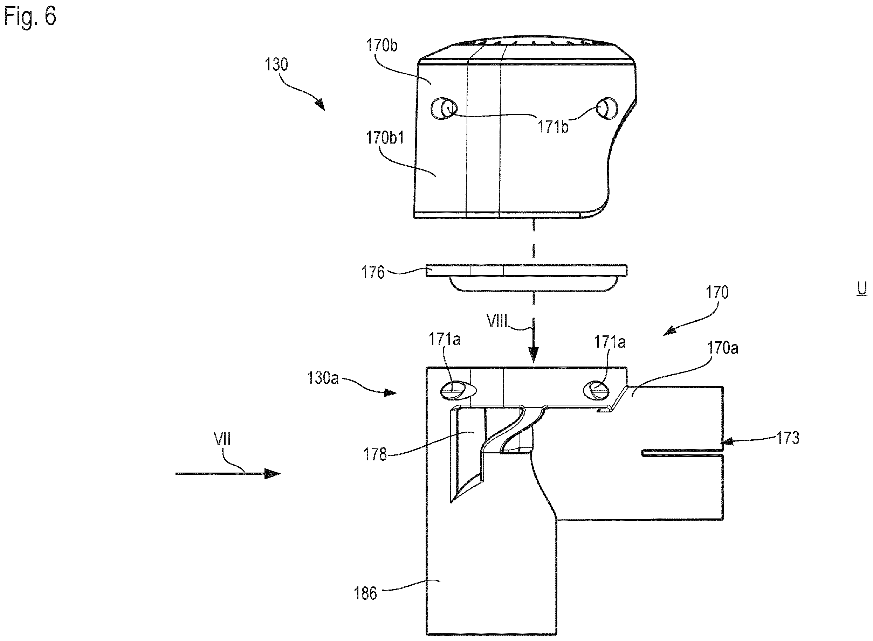

In Figur 6 ist eine Explosions-Aufrissansicht einer alternativen Ausführungsform eines Exspirationsventils 130 dargestellt. Die zweite Ausführungsform des Exspirationsventils 130 wird nachfolgend nur insoweit beschrieben werden, als sie sich von der ersten Ausführungsform eines Exspirationsventils gemäß den Figuren 4 und 5 unterscheidet, auf deren Beschreibung ansonsten auch zur Erläuterung der zweiten Ausführungsform des Exspirationsventils 130 verwiesen wird. Gleiche und funktionsgleiche Bauteile und Bauteilabschnitte der zweiten Ausführungsform des Exspirationsventils 130 sind mit gleichen Bezugszeichen versehen wie in der ersten Ausführungsform der Figuren 4 und 5, jedoch erhöht um die Zahl 100.

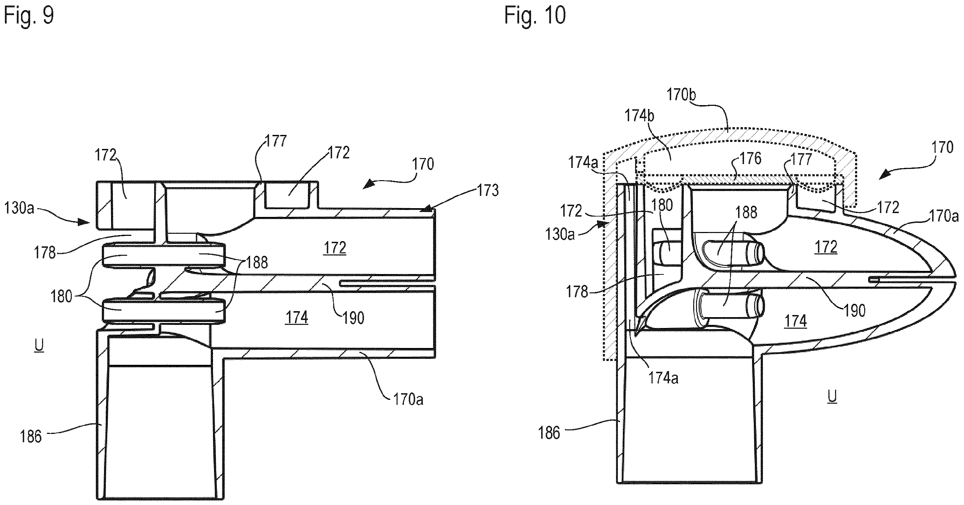

Während bei der ersten Ausführungsform nur der Inspirationskanal 74 im Exspirationsventil 30 abgewinkelt ausgebildet ist und der Exspirationskanal 72 in gerader Linie durch das Gehäuse 70 auf den Membran-Ventilkörper 76 zu verläuft, ist im Exspirationsventil 130 der zweiten Ausführungsform auch der Exspirationskanal 172 abgewinkelt ausgeführt. Bevorzugt sind der Inspirationskanal 174 und der Exspirationskanal 172 jeweils um 90° abgewinkelt, und zwar längs ihres Verlaufs vom Beatmungsschlauch weg besonders bevorzugt in entgegengesetzte Richtungen (siehe Figuren 9 und 10).

Der Deckel 170b des Exspirationsventils 130 weist eine Schürze 170b1 auf, welche im fertig montierten Zustand die Fenster 178 bedeckt, welche den Exspirationskanal 172 zur Außenumgebung U des Exspirationsventils 130 öffnen. Dabei ist die Schürze 170b1 mit Abstand von dem die Fenster 178 aufweisenden Grundkörper 170a angeordnet, um eine Strömung von exspiratorischem Atemgas in die Außenumgebung zu ermöglichen. Die die Fenster 878 bedeckende Schürze 170b1 wirkt als Tröpfchen-Spritzschutz gegen ein unkontrolliertes Mitführen und Austreten von Tröpfchen im exspiratorischem Atemgas und wirkt außerdem geräuschmindernd.

Durch Ausbildung von Verrastungsvorsprüngen 171a an einem der Bauteile aus Grundkörper 170a und Deckel 170b, in dargestellten Beispiel am Grundkörper 170a, sowie von komplementären Verrastungsausnehmungen 171b am jeweils anderen Bauteil aus Grundkörper 170a und Deckel 170b, in dargestellten Beispiel am Deckel 170b, lässt sich der Deckel 170b einfach durch selbsttätige formschlüssige Verrastung am Grundkörper 170a in der korrekten Relativposition dauerhaft anordnen. Die Schürze 170b1 dient daher auch als Orientierungshilfe bei der Anordnung des Deckels 170b am Grundkörper 170a, da der Deckel 170b sich aufgrund der Schürze 170b1 an dem grob aus einem rechtwinkligen Rohrstück gebildeten Grundkörper 170a nur in einer einzigen Relativposition sinnvoll anordnen lässt.

Bevorzugt sind die Verrastungsausnehmungen 171b am Deckel 170b diesen durchsetzend ausgebildet, sodass sich durch Betrachtung des Exspirationsventils 130 von außen der korrekte Sitz des Deckels 170b am Grundkörper 170a visuell in einfacher Weise überprüfen lässt.

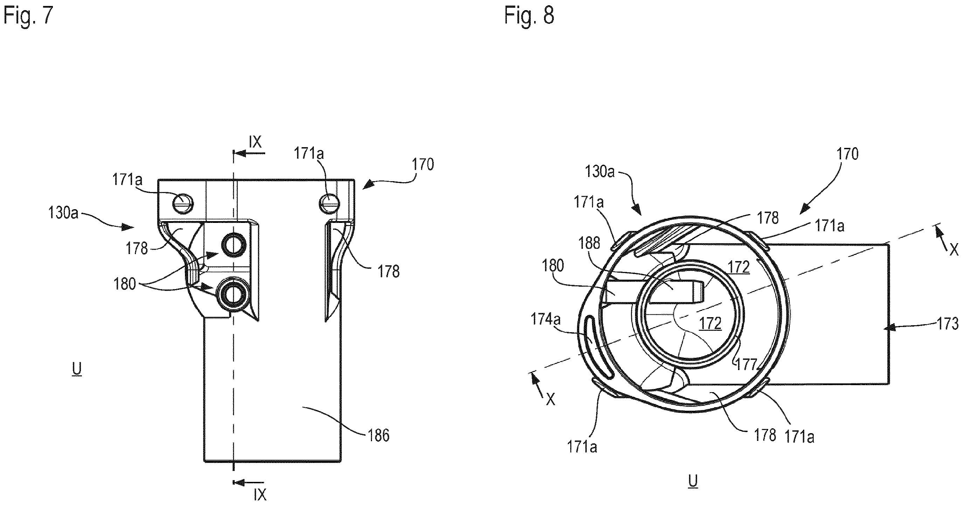

Figur 7 zeigt eine Ansicht des Gehäuse-Grundkörpers 170a von hinten, also entlang des zur Zeichenebene von Figur 6 parallelen Pfeils VII von Figur 6.

Zu erkennen ist in Figur 7, dass die äußeren Anschlussstutzen 180 im Bereich eines Fensters 178 am Grundkörper 170a angeordnet und zum Anschluss von flexiblen Schläuchen ausgebildet sind. Die Anschlussstutzen 180 sind also anders als in der ersten Ausführungsform bei Annäherung vom Beatmungsschlauch her nicht vor dem den Ventilsitz 177 aufweisenden und den Membran-Ventilkörper 176 tragenden Austrittsbereich 130a des Exspirationsventils 130 herausgeführt, sondern befinden sich in dem Austrittsbereich 130a, und zwar bevorzugt auf der von einer Anschlussformation 173 zur Anschluss eines zum Patienten führenden Beatmungsschlauchs abgewandten Seite des Austrittsbereich 130a.

Der Austrittsbereich 130a ist vorliegend gekennzeichnet durch eine konzentrische oder/und kollineare oder parallele Anordnung von Abschnitten des Exspirationskanals 172, welche durch den Membran-Ventilkörper 176 voneinander strömungsmechanisch trennbar sind. In der in den Figuren 6 bis 10 dargestellten zweiten Ausführungsform des Exspirationsventils 130 sind diese Abschnitte des Exspirationskanals 172 konzentrisch und kollinear angeordnet.

Die äußeren Anschlussstutzen 180 sind, bevorzugt einstückig, mit inneren Anschlussstutzen 188 verbunden. Die äußeren und die inneren Anschlussstutzen 180 bzw. 188 bilden jeweils ein geradliniges Rohrstück, welches vorteilhaft parallel zum Verlauf des exspiratorischen Strömungspfads im Bereich der Verbindung des Grundkörpers 170a mit einem Beatmungsschlauch, also im Bereich der Anschlussformation 173, verläuft. Hierdurch kann ein gekrümmter oder sogar abgewinkelter Verlauf der die inneren Anschlussstutzen 188 mit einem Differenzdruck-Durchflusssensor verbindenden Leitungsanordnung (entsprechend der in der ersten Ausführungsform dargestellten Leitungsanordnung 50) weitgehend vermieden werden. Dieser stärker oder bevorzugt vollständig geradlinige Verlauf der Leitungsanordnung im Gehäuse 170 des Exspirationsventils 130 verringert den durch die Leitungsanordnung bewirkten Strömungswiderstand erheblich, sodass der durch den Druckunterschied im Differenzdruck-Durchflusssensor ermittelte Atemgasfluss genauer bestimmbar ist.

In Figur 8 ist der radial außerhalb der konzentrisch angeordneten Abschnitte des Exspirationskanals 172 einstückig am Grundkörper 170a ausgebildete Steuerkanal 174a zu erkennen, durch welchen der Druck im Steuerraum 174b (siehe Figur 10) erhöht werden kann.

Figur 9 ist eine Schnittansicht des Grundkörpers 170a entlang der Schnittebene IX-IX in Figur 7. Bevorzugt ist der Grundkörper 170a, wie im Übrigen ebenso der Deckel 170b, als einstückiges Spritzgussbauteil ausgebildet, wobei auch die inneren und die äußeren Anschlussstutzen 188 bzw. 180 vorzugsweise einstückig mit dem übrigen Grundkörper 170a ausgebildet sind.

Wie in Figur 9 weiter zu erkennen ist, sind die inneren Anschlussstutzen 188 in beiden Kanälen 172 und 174 bevorzugt in einem Bereich angeordnet, in welchen die Kanäle 172 und 174 abgewinkelt sind, in welchem also der Exspirationskanal 172 zum Membran-Ventilkörper 176 hin abgewinkelt ist und in welchem der Inspirationskanal 174 zur Anschlussformation 186 hin abgewinkelt ist.

Figur 10 ist eine Schnittansicht entlang der Schnittebene X-X von Figur 8. Figur 10 zeigt insbesondere die Ausbildung des Steuerkanals 174a im Grundkörper 170a.

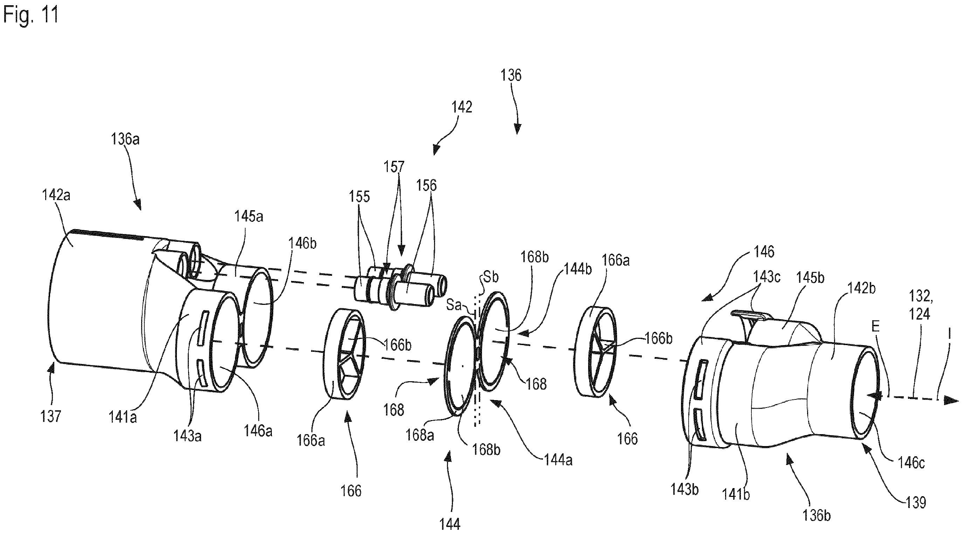

In Figur 11 ist eine zweite Ausführungsform der Ventilbaugruppe 136 dargestellt. Die zweite Ausführungsform der Ventilbaugruppe 136 wird nachfolgend nur insofern erläutert werden, als sie sich von der ersten Ausführungsform der Ventilbaugruppe 36 unterscheidet, auf deren Beschreibung ansonsten auch zur Erläuterung der zweiten Ausführungsform verwiesen wird. Gleiche und funktionsgleiche Bauteile und Bauteilabschnitte wie in der ersten Ausführungsform sind in der zweiten Ausführungsform mit gleichen Bezugszeichen versehen, jedoch erhöht um die Zahl 100.

Im Gegensatz zu ersten Ausführungsform sind die Kanallumina 146a und 146b in dem Abschnitt, in welchem die Ventilanordnung 144 aufgenommen ist, durch zwei zwar körperlich miteinander verbundene, jedoch jeweils für sich ausgebildete Rohrabschnitte 141a, 145a, 141b und 145b ausgebildet. Die Rohrabschnitte 141a, 145a, 141b und 145b, also die distalen Rohrabschnitte 141a und 154 sowie die proximalen Rohrabschnitte 141b und 145b, sind längs ihres Verlaufs trennbar, da sie abschnittsweise am distalen Gehäusebauteil 142a und am proximalen Gehäusebauteil 142b ausgebildet sind. Die Gehäusebauteile 142a und 142b sind miteinander verrastbar.

Hierzu ist an einem Gehäusebauteil, vorliegend ist dies das distale Gehäusebauteil 142a, wenigstens ein Verrastungsvorsprung 143a ausgebildet, und ist am jeweils anderen Gehäusebauteil, im dargestellten Beispiel ist dies das proximale Gehäusebauteil 142b, wenigstens eine Verrastungsausnehmung 143b ausgebildet, wobei der wenigstens eine Verrastungsvorsprung 143a mit der wenigstens einen Verrastungsausnehmung 143b in einen Verrastungseingriff bringbar ist. Wiederum ist, wie schon beim Expansionsventil 130 der zweiten Ausführungsform, die Verrastungsausnehmung 143b an dem bei hergestelltem Verrastungseingriff außenliegenden Gehäusebauteilabschnitt ausgebildet und durchsetzt den Gehäusebauteilabschnitt in Dickenrichtung vollständig, sodass eine ordnungsgemäße Herstellung des Verrastungseingriffs in einfacher Weise visuell überprüfbar ist. Eine der Verrastungsformationen aus Verrastungsvorsprung 143a und Verrastungsausnehmung 143b kann an eigens dafür vorgesehenen Verbindungsbacken 143c ausgebildet sein, um eine für eine dauerhafte Verbindung ausreichende Überlappung der Gehäusebauteile 142a und 142b längs des inspiratorischen und des exspiratorischen Strömungspfads 124 bzw. 132 gewährleisten zu können. Vorliegend ist die wenigstens eine Verrastungsausnehmung 143b an solchen Verbindungsbacken 143c ausgebildet.

Die Ventilkörper 168 umfassen einen gemeinsamen Ventilkörperahmen 168a und relativ zum Ventilkörperrahmen 168a bewegliche Ventilkörperblätter 168b. Bevorzugt sind die Ventilkörperblätter 168b einstückig mit dem Ventilkörperrahmen 168a ausgebildet, beispielsweise durch Spritzgießen oder durch Ausstanzen aus einem ebenen Materialbogen. Der Ventilkörperrahmen 168a wird bei der Montage der Ventilbaugruppe 136 zwischen den miteinander verbundenen Rohrabschnitten 141a und 145a auf der baulich distalen Seite sowie 141b und 145b auf der baulich proximalen Seite angeordnet und bei Herstellung des Verrastungseingriffs des wenigstens einen Verrastungsvorsprung 143a mit der wenigstens einen Verrastungsausnehmung 143b zwischen den distalen und proximalen Rohrabschnitten geklemmt. Die den Ort der Anordnung des Ventilkörperrahmens 168a längs der Strömungspfade 124 und 132 überlappenden Verbindungsbacken 143c verhindern eine Verlagerung des Ventilkörperrahmens 168a relativ zu den miteinander fluchtenden distalen und proximalen Rohrabschnitten 141a, 145a, 141b und 145b und verhindert damit auch eine Verlagerung der Ventilkörperblätter 168b. Die Größe der Ventilblätter 168b ist derart bemessen, dass sie in den Kanallumina 146a bzw. 146b ungehindert um ihre Schwenkachsen Sa bzw. Sb schwenken können. Durch die aufgrund der einstückigen Ausbildung der Ventilkörper 168 bestehenden Materialbrücke zwischen den Ventilkörperblätter 168b und dem Ventilkörperrahmen 168a sind die Ventilkörperblätter 168b in die in Figur 11 dargestellte Schließstellung vorgespannt. Die Schwenkachsen Sa und Sb sind daher in der zweiten Ausführungsform Biegeachsen Sa und Sb, um welche die Ventilkörperblätter 168b durch Biegung aus ihrer Schließstellung auslenkbar sind.

Die zu Erleichterung der Fertigung und Montage identisch ausgebildeten Ventilsitze 166, welche aus demselben Material wie das Ventilgehäuse 142 hergestellt sein können, umfassen jeweils einen Ringabschnitt 166a, dessen Außenumfang dem Innenumfang des ihn aufnehmenden Rohrabschnitts 141a bzw. 145b soweit entspricht, dass der Ventilsitz 166 in seinen Rohrabschnitt axial eingeführt werden kann und dort reibschlüssig aufgrund der zwischen dem Außenumfang des Ringabschnitts 166a und der Innenfläche des zugeordneten Rohrabschnitts wirkenden Reibung an Ort und Stelle gehalten ist. Alternativ oder zusätzlich kann der Ventilsitz 166 auch in seinen Rohrabschnitt eingeklebt sein, was jedoch nicht bevorzugt ist, um Ausdünstungen von Klebstoff in einem Atemgas-Strömungspfad 124 und 132 zu vermeiden. Eine alternative Festlegung des Ventilsitzes 166 in seinem Rohrabschnitt durch Ultraschallverschweißung ist möglich.

Die Ventilsitze 166 weisen radial innerhalb des Ringabschnitts 166a eine Speichenstruktur 166b auf, welche einerseits den durchströmbaren Querschnitt des Ringabschnitts 166a nur unwesentlich verringert und welche andererseits als Abstützstruktur für den ihr längs des jeweiligen Strömungspfads 124 bzw. 132 unmittelbar benachbarten Ventilkörperblatts 166b dient und so eine Auslenkung des Ventilkörperblatts 166b aus seiner in Figur 11 gezeigten Schließstellung nur in einer Richtung gestattet.

Die inneren Anschlussstutzen 155 und die äußeren Anschlussstutzen 156 sind jeweils an einem, vorzugsweise einstückig ausgebildeten, Rohrstück 157 ausgebildet, welche mit ihrer Rohrachse parallel zu dem Verlauf des inspiratorischen Strömungspfads 124 und des exspiratorischen Strömungspfads 132 im Bereich des distalen Auslassendbereichs 137 der Ventilbaugruppe 136 angeordnet. Somit ist auch auf Seiten der Ventilbaugruppe 136 sichergestellt, dass eine in einem Beatmungsschlauch zwischen dem Exspirationsventil 130 oder 30 verlaufende Leitungsanordnung (entspricht der Leitungsanordnung 50 die ersten Ausführungsform) ohne weitere Umlenkung bezüglich des Verlaufs der Leitungsanordnung im Beatmungsschlauch im Bereich eines Austritts aus dem Inneren des Gehäuses 142 in den Außenbereich des Gehäuses 142 herausgeführt werden kann. Auch diese Vermeidung einer Umlenkung der Leitungsanordnung im Austrittsbereich aus dem Gehäuse 142 verringert den Strömungswiderstand der Leitungsanordnung und erhöht somit die Genauigkeit einer Bestimmung des Atemgasflusses durch einen auf der baulich proximalen Seite an das Gehäuse 142 angeschlossenen Differenzdruck-Durchflusssensor.

## Patentansprüche

1. Atemgas-Ventilbaugruppe (36) für eine Beatmungsleitungsanordnung (20) einer Beatmungsvorrichtung (10) zur wenigstens unterstützenden künstlichen Beatmung eines Patienten (P), wobei die Atemgas-Ventilbaugruppe (36) ein Gehäuse (42) mit einer bidirektional von Atemgas durchströmbaren Kanalanordnung (46) aufweist, welche Kanalanordnung (46) einen inspiratorischen (24) und einen exspiratorischen Strömungspfad (32) aufweist, wobei der inspiratorische Strömungspfad (24) der Kanalanordnung (46) von einem distalen Einlassendbereich (37) zu einem proximalen Auslassendbereich (39) der Atemgas-Ventilbaugruppe (36) verläuft und wobei der exspiratorische Strömungspfad (32) der Kanalanordnung (46) von einem proximalen Einlassendbereich (39) zu einem distalen Auslassendbereich (37) der Atemgas-Ventilbaugruppe (36) verläuft, wobei die Atemgas-Ventilbaugruppe (36) zwischen ihren jeweiligen Einlass- und Auslassendbereichen (37, 39) eine Ventilanordnung (44) aufweist, welche wenigstens verstellbar ist zwischen einer ersten Betriebsstellung, in welcher der inspiratorische Strömungspfad (24) zur Durchströmung freigegeben und der exspiratorische Strömungspfad (32) für eine Durchströmung gesperrt ist, und einer zweiten Betriebsstellung, in welcher der exspiratorische Strömungspfad (32) zur Durchströmung freigegeben und der inspiratorische Strömungspfad (24) für eine Durchströmung gesperrt ist, wobei der inspiratorische und der exspiratorische Strömungspfad (24, 32) auf wenigstens einer baulichen Seite (36a) der Atemgas-Ventilbaugruppe (36) bezüglich der Ventilanordnung (44) in körperlich voneinander getrennten Strömungsführungsabschnitten (46a, 46b) der Kanalanordnung (46) verlaufen, **dadurch gekennzeichnet, dass** die Ventilanordnung (44) eine Ventilkörperanordnung aufweist, umfassend einen Ventilkörperrahmen und am Ventilkörperrahmen beweglich gehalterte Ventilkörper (68), wobei die Ventilanordnung (44) ein die Durchströmbarkeit des inspiratorischen Strömungspfads (24) steuerndes inspiratorisches Ventil (44a) und ein die Durchströmbarkeit des exspiratorischen Strömungspfads (32) steuerndes exspiratorisches Ventil (44b) aufweist, wobei das inspiratorische Ventil (44a) und das exspiratorische Ventil (44b) in ihre den jeweiligen Strömungspfad (24, 32) gegen eine Durchströmung sperrende Sperrstellung vorgespannt sind, wobei das inspiratorische Ventil (44a) durch Erhöhung des Drucks des inspiratorischen Atemgases gegen seine Vorspannung in die Sperrstellung öffenbar ist, um dem Patienten inspiratorisches Atemgas zuzuleiten und wobei nach einem Abklingen des Überdrucks im inspiratorischen Atemgas ein Überdruck des exspiratorischen Atemgases nicht nur eine zusätzliche Schließkraft auf das inspiratorische Ventil ausübt, sondern gleichzeitig das exspiratorische Ventil (44b) gegen seine Vorspannung in die Sperrstellung öffnet.

2. Atemgas-Ventilbaugruppe (36) nach Anspruch 1,
**dadurch gekennzeichnet, dass** der inspiratorische und der exspiratorische Strömungspfad (24, 34) auf einer baulichen Seite (36a) der Atemgas-Ventilbaugruppe (36) bezüglich der Ventilanordnung (44) in körperlich voneinander getrennten Kanalabschnitten (46a, 46b) verlaufen und auf der anderen baulichen Seite (36b) der Atemgas-Ventilbaugruppe (36) bezüglich der Ventilanordnung (44) in einem gemeinsamen Strömungsführungsabschnitt (46c) der Kanalanordnung (46) verlaufen.

3. Atemgas-Ventilbaugruppe (36) nach Anspruch 1 oder 2,
**dadurch gekennzeichnet, dass** die körperlich voneinander getrennten Kanalabschnitte (46a, 46b) auf der einen baulichen Seite (36a) der Atemgas-Ventilbaugruppe (36) bezüglich der Ventilanordnung (44) durch einen Mehrlumenkanal gebildet sind.

4. Atemgas-Ventilbaugruppe (36) nach einem der vorhergehenden Ansprüche, unter Einbeziehung des Anspruchs 2,
**dadurch gekennzeichnet, dass** ein sowohl den proximalen Einlassendbereich als auch den proximalen Auslassendbereich aufweisender proximaler Endbereich als proximale Anschlussformation zum Anschluss einer proximalen Leitungskomponente (48) ausgebildet ist.

5. Atemgas-Ventilbaugruppe (36) nach einem der vorhergehenden Ansprüche, **dadurch gekennzeichnet, dass** die Atemgas-Ventilbaugruppe (36) eine Leitungsanordnung (50) aufweist, welche mit wenigstens einem körperlich von der Atemgas führenden Kanalanordnung (46) getrennten Lumen ausgebildet ist, wobei ein innerer Teil (54) der Leitungsanordnung (50) derart innerhalb der Kanalanordnung (46) verläuft, dass wenigstens ein Abschnitt einer Außenseite des inneren Teils (54) der Leitungsanordnung (50) im Betrieb von Atemgas benetzbar ist, oder/und wobei ein äußerer Teil (52) der Leitungsanordnung (50) derart außerhalb eines von Atemgas durchströmbaren Kanalvolumens der Kanalanordnung (46) verläuft, dass ein äußerer Teil (52) der Leitungsanordnung (50) im Betrieb von Atemgas nicht benetzbar ist.

6. Atemgas-Ventilbaugruppe (36) nach Anspruch 5,
**dadurch gekennzeichnet, dass** der innere Teil (54) der Leitungsanordnung (50) und wenigstens ein Abschnitt des äußeren Teils (52) der Leitungsanordnung (50) zwischen den distalen Endbereichen (37) der Atemgas-Ventilbaugruppe (36) und der Ventilanordnung (44) ausgebildet sind.

7. Atemgas-Ventilbaugruppe (36) nach Anspruch 5 oder 6,
**dadurch gekennzeichnet, dass** die Leitungsanordnung (50) das Gehäuse (42) der Atemgas-Ventilbaugruppe (36) zwischen den distalen Endbereichen (37) der Atemgas-Ventilbaugruppe (36) und der Ventilanordnung (44) durchsetzt.

8. Atemgas-Ventilbaugruppe (36) nach einem der vorhergehenden Ansprüche, **dadurch gekennzeichnet, dass** die Ventilkörper (668) Ventilkörperblätter zur Bildung von Blattventilen sind.

9. Beatmungsvorrichtung (10) zur wenigstens unterstützend künstlichen Beatmung eines lebenden Patienten, umfassend:
- eine Atemgasquelle (12),
- eine Beatmungsleitungsanordnung (20), um inspiratorisches Atemgas von der Atemgasquelle (12) zu einer patientenseitigen, proximalen Atemgas-Auslassöffnung (62) und um exspiratorisches Atemgas von einer proximalen Atemgas-Einlassöffnung (62) weg zu leiten,
- eine Druckveränderungsvorrichtung (13b) zur Veränderung des Drucks des Atemgases in der Beatmungsleitungsanordnung (20),
- eine Steuervorrichtung (14) zum Betrieb der Atemgasquelle (12) oder/und der Druckveränderungsvorrichtung (13b) sowie
- eine Atemgas-Ventilbaugruppe (36) nach einem der vorhergehenden Ansprüche.

10. Beatmungsvorrichtung (10) nach Anspruch 9,
**dadurch gekennzeichnet, dass** die Beatmungsleitungsanordnung (20) einen Multi-Lumen-Schlauch (22), insbesondere Bi-Lumen-Schlauch (22), mit einer wenigstens längs eines Längsabschnitts des Multi-Lumen-Schlauchs (22) in wenigstens einem Lumen geführten Leitungsanordnung (50) umfasst.

11. Beatmungsvorrichtung (10) nach Anspruch 10,
**dadurch gekennzeichnet, dass** die Leitungsanordnung (50) eine den Multi-Lumen-Schlauch (22) zur Außenumgebung (U) hin begrenzende Schlauchwand durchsetzt.

12. Beatmungsvorrichtung (10) nach einem der Ansprüche 10 oder 11,
**dadurch gekennzeichnet, dass** der Multi-Lumen-Schlauch (22) die Atemgas-Ventilbaugruppe (36) mit einem distalen Exspirationsventil (30) atemgasleitend verbindet.

13. Beatmungsvorrichtung (10) nach Anspruch 12,
**dadurch gekennzeichnet, dass** das Exspirationsventil (30) ein Membranventil mit einer von einem Ventilsitz (77) abhebbaren Ventilmembran (76) ist, wobei die Ventilmembran (76) auf der dem Multi-Lumen-Schlauch (22) zugewandten Seite mit exspiratorischem Atemgas beaufschlagbar ist und wobei die Ventilmembran (76) auf der dem Multi-Lumen-Schlauch (22) abgewandten Seite mit inspiratorischem Atemgas beaufschlagbar ist.

14. Beatmungsvorrichtung (10) nach einem der Ansprüche 10 bis 13,
**dadurch gekennzeichnet, dass** zwischen dem proximalen Längsende des Multi-Lumen-Schlauchs (22) einerseits und der proximalen Atemgas-Auslassöffnung (62) oder/und der proximalen Atemgas-Einlassöffnung (62) andererseits eine Spirometrie-Sensoranordnung (48) angeordnet ist, mit welcher die Leitungsanordnung (50) verbunden ist.

15. Beatmungsvorrichtung (10) nach Anspruch 14,
**dadurch gekennzeichnet, dass** die Spirometrie-Sensoranordnung (48) eine Durchflusssensoranordnung (48), insbesondere eine Differenzdruck-Durchflusssensoranordnung (48), zur Erfassung des inspiratorischen oder/und des exspiratorischen Atemgasflusses ist.

## Claims

1. Respiratory gas valve module (36) for a ventilation line arrangement (20) of a ventilation device (10) for at least supportive artificial ventilation of a patient (P), where the respiratory gas valve module (36) exhibits a housing (42) with a duct arrangement (46) through which respiratory gas is able to flow bidirectionally, which duct arrangement (46) exhibits an inspiratory (24) and an expiratory flow path (32), where the inspiratory flow path (24) of the duct arrangement (46) proceeds from a distal inlet end region (37) to a proximal outlet end region (39) of the respiratory gas valve module (36) and where the expiratory flow path (32) of the duct arrangement (46) proceeds from a proximal inlet end region (39) to a distal outlet end region (37) of the respiratory gas valve module (36), where the respiratory gas valve module (36) exhibits between its respective inlet and outlet end regions (37, 39) a valve arrangement (44) which at least is adjustable between a first operating position in which the inspiratory flow path (24) is released for flowthrough and the expiratory flow path (32) is blocked for a flowthrough and a second operating position in which the expiratory flow path (32) is released for flowthrough and the inspiratory flow path (24) is blocked for a flowthrough, where the inspiratory and the expiratory flow path (24, 32) proceed on at least one structural side (36a) of the respiratory gas valve module (36) relative to the valve arrangement (44) in flow-conducting sections (46a, 46b) physically separated from one another of the duct arrangement (46),
**Characterized in that** the valve arrangement (44) exhibits a valve body arrangement comprising a valve body frame and valve bodies (68) retained moveably at the valve body frame, where the valve arrangement (44) exhibits an inspiratory valve (44a) which controls the flowthrough ability of the inspiratory flow path (24) and an expiratory valve (44b) which controls the flowthrough ability of the expiratory flow path (32), where the inspiratory valve (44a) and the expiratory valve (44b) are pre-tensioned in a blocked position blocking the respective flow path (24, 32) against a flowthrough, where the inspiratory valve (44a) is openable against its pre-tensioning in the blocked position through an increase in the pressure of the inspiratory respiratory gas, in order to supply inspiratory respiratory gas to the patient and where after subsidence of the overpressure in the inspiratory respiratory gas an overpressure of the expiratory respiratory gas not only exerts an additional closing force on the inspiratory valve, but at the same time opens the expiratory valve (44b) against its pre-tensioning in the blocked position.

2. Respiratory gas valve module (36) according to Claim 1,
**Characterized in that** the inspiratory and the expiratory flow path (24, 34) on one structural side (36a) of the respiratory gas valve module (36) relative to the valve arrangement (44) proceed in duct sections (46a, 46b) physically separated from one another and on the other structural side (36b) of the respiratory gas valve module (36) relative to the valve arrangement (44) proceed in a common flow-conducting section (46c) of the duct arrangement (46).

3. Respiratory gas valve module (36) according to Claim 1 or 2,
**Characterized in that** the duct sections (46a, 46b) physically separated from one another on the one structural side (36a) of the respiratory gas valve module (36) relative to the valve arrangement (44) are formed through a multi-lumen duct.

4. Respiratory gas valve module (36) according to one of the preceding Claims, in consideration of Claim 2,
**Characterized in that** a proximal end region exhibiting both the proximal inlet end region and the proximal outlet end region is configured as a proximal connector formation for connecting a proximal line component (48).

5. Respiratory gas valve module (36) according to one of the preceding Claims, **Characterized in that** the respiratory gas valve module (36) exhibits a line arrangement (50) which is configured with at least one lumen physically separated from the respiratory gas conducting duct arrangement (46), where an inner part (54) of the line arrangement (50) proceeds in such a manner within the duct arrangement (46) that at least one section of an outer side of the inner part (54) of the line arrangement (50) is wettable by respiratory gas during operation, and/or where an outer part (52) of the line arrangement (50) proceeds in such a manner outside a duct volume through which respiratory gas is able to flow of the duct arrangement (46) that an outer part (52) of the line arrangement (50) is not wettable by respiratory gas during operation.

6. Respiratory gas valve module (36) according to Claim 5,
**Characterized in that** the inner part (54) of the line arrangement (50) and at least one section of the outer part (52) of the line arrangement (50) are formed between the distal end regions (37) of the respiratory gas valve module (36) and the valve arrangement (44).

7. Respiratory gas valve module (36) according to Claim 5 or 6,
**Characterized in that** the line arrangement (50) penetrates through the housing (42) of the respiratory gas valve module (36) between the distal end regions (37) of the respiratory gas valve module (36) and the valve arrangement (44).

8. Respiratory gas valve module (36) according to one of the preceding Claims, **Characterized in that** the valve bodies (668) are valve body reeds for forming reed valves.

9. Ventilation device (10) for at least supportively artificial ventilation of a living patient, comprising:
- A respiratory gas source (12),
- A ventilation line arrangement (20) in order to guide inspiratory respiratory gas from the respiratory gas source (12) to a patient-side, proximal respiratory gas outlet aperture (62) and expiratory respiratory gas away from a proximal respiratory gas inlet aperture (62),
- A pressure modification device (13b) for modifying the pressure of the respiratory gas in the ventilation line arrangement (20),
- A control device (14) for operating the respiratory gas source (12) and/or the pressure modification device (13b), and
- A respiratory gas valve module (36) according to one of the preceding Claims.

10. Ventilation device (10) according to Claim 9,
**Characterized in that** the ventilation line arrangement (20) comprises a multi-lumen hose (22), in particular bi-lumen hose (22), with a line arrangement (50) which is guided in at least one lumen at least along one longitudinal section of the multi-lumen hose (22).

11. Ventilation device (10) according to Claim 10,
**Characterized in that** the line arrangement (50) penetrates through a hose wall which bounds the multi-lumen hose (22) towards the external environment (U).

12. Ventilation device (10) according to one of the Claims 10 or 11,
**Characterized in that** the multi-lumen hose (22) connects the respiratory gas valve module (36) with a distal expiration valve (30) in a respiratory gasconducting manner.

13. Ventilation device (10) according to Claim 12,
**Characterized in that** the expiration valve (30) is a membrane valve with a valve membrane (76) which can be raised from a valve seat (77), where expiratory respiratory gas can be applied to the valve membrane (76) on the side facing towards the multi-lumen hose (22) and where inspiratory respiratory gas can be applied to the valve membrane (76) on the side facing away from the multi-lumen hose (22).

14. Ventilation device (10) according to one of the Claims 10 bis 13,
**Characterized in that** between the proximal longitudinal end of the multi-lumen hose (22) on the one hand and the proximal respiratory gas outlet aperture (62) and/or the proximal respiratory gas inlet aperture (62) on the other hand there is arranged a spirometry sensor arrangement (48) with which the line arrangement (50) is connected.

15. Ventilation device (10) according to Claim 14,
**Characterized in that** the spirometry sensor arrangement (48) is a flow sensor arrangement (48), in particular a differential pressure flow sensor arrangement (48), for acquiring the inspiratory and/or the expiratory respiratory gas flow.

## Revendications

1. Agencement de soupape de gaz respiratoire (36) pour un agencement de conduits respiratoires (20) d'un Dispositif de ventilation (10) pour au moins la ventilation artificielle assistée d'un patient (P), dans lequel l'agencement de soupape de gaz respiratoire (36) présente un boîtier (42) avec un agencement de canaux (46) pouvant être traversé de manière bidirectionnelle par du gaz respiratoire, lequel agencement de canaux (46) présente un chemin d'écoulement inspiratoire (24) et un chemin d'écoulement expiratoire (32), dans lequel le chemin d'écoulement inspiratoire (24) de l'agencement de canaux (46) s'étend d'une zone d'extrémité d'entrée distale (37) à une zone d'extrémité de sortie proximale (39) de l'agencement de soupape de gaz respiratoire (36) et dans lequel le chemin d'écoulement expiratoire (32) de l'agencement de canaux (46) s'étend d'une zone d'extrémité d'entrée proximale (39) à une zone d'extrémité de sortie distale (37) de l'agencement de soupape de gaz respiratoire (36), dans lequel l'agencement de soupape de gaz respiratoire (36) comprend, entre ses zones d'extrémité d'entrée et de sortie respectives (37, 39), un agencement de soupape (44) qui est réglable au moins entre une première position de fonctionnement, dans laquelle le chemin d'écoulement inspiratoire (24) est libéré pour l'écoulement et le chemin d'écoulement expiratoire (32) est bloqué pour l'écoulement, et une deuxième position de fonctionnement dans laquelle le chemin d'écoulement expiratoire (32) est libéré pour l'écoulement et le chemin d'écoulement inspiratoire (24) est bloqué pour l'écoulement, dans lequel le chemin d'écoulement inspiratoire et le chemin d'écoulement expiratoire (24, 32) s'étendent sur au moins un côté de construction (36a) de l'agencement de soupape de gaz respiratoire (36) par rapport à l'agencement de soupape (44) dans des sections de guidage d'écoulement (46a, 46b) de l'agencement de canaux (46) physiquement séparées les unes des autres,
**caractérisé en ce que** l'agencement de soupape (44) présente un agencement de corps de soupape, comprenant un cadre de corps de soupape et des corps de soupape (68) maintenus de manière mobile sur le cadre de corps de soupape, dans lequel l'agencement de soupape (44) présente une soupape inspiratoire (44a) commandant l'aptitude à l'écoulement du chemin d'écoulement inspiratoire (24) et une soupape expiratoire (44b) commandant l'aptitude à l'écoulement du chemin d'écoulement expiratoire (32), dans lequel la soupape inspiratoire (44a) et la soupape expiratoire (44b) sont précontraintes dans leur position de blocage bloquant le passage de l'écoulement dans le chemin d'écoulement respectif (24, 32), dans lequel la soupape inspiratoire (44a) peut être ouverte en augmentant la pression du gaz respiratoire inspiratoire à l'encontre de sa précontrainte dans la position de blocage afin d'amener le gaz respiratoire inspiratoire au patient et, dans lequel après une diminution de la surpression dans le gaz respiratoire inspiratoire, une surpression du gaz respiratoire expiratoire n'exerce pas seulement une force de fermeture supplémentaire sur la soupape inspiratoire, mais ouvre en même temps la soupape expiratoire (44b) à l'encontre de sa précontrainte dans la position de blocage.

2. Agencement de soupape de gaz respiratoire (36) selon la revendication 1, **caractérisé en ce que** les chemins d'écoulement inspiratoire et expiratoire (24, 34) sur un côté de construction (36a) de l'agencement de soupape de gaz respiratoire (36) s'étendent par rapport à l'agencement de soupape (44) dans des sections de canal physiquement séparées (46a, 46b) et, de l'autre côté de construction (36b) de l'agencement de soupapes de gaz respiratoire (36) s'étendent par rapport à l'agencement de soupape (44), dans une section de guidage d'écoulement (46c) commune de l'agencement de canaux (46).

3. Agencement de soupape de gaz respiratoire (36) selon la revendication 1 ou 2,
**caractérisé en ce que** les sections de canal (46a, 46b) physiquement séparées les unes des autres sont formées par un canal à plusieurs lumières sur l'un des côtés de construction (36a) de l'agencement de soupape de gaz respiratoire (36) par rapport à l'agencement de soupape (44).

4. Agencement de soupape de gaz respiratoire (36) selon l'une des revendications précédentes, en incluant la revendication 2,
**caractérisé en ce qu'**une zone d'extrémité proximale présentant à la fois la zone d'extrémité d'entrée proximale et la zone d'extrémité de sortie proximale est réalisée sous forme de formation de raccordement proximale pour le raccordement d'un composant de conduite proximal (48).

5. Agencement de soupape de gaz respiratoire (36) selon l'une des revendications précédentes,
**caractérisé en ce que** l'agencement de soupape de gaz respiratoire (36) comprend un agencement de conduit (50) formé avec au moins une lumière physiquement séparée de l'agencement de canaux de gaz respiratoire (46), dans lequel une partie intérieure (54) de l'agencement de conduit (50) s'étend de telle sorte à l'intérieur de l'agencement de canaux (46), qu'au moins une section d'un côté extérieur de la partie intérieure (54) de l'agencement de conduit (50) peut être mouillée par le gaz respiratoire pendant le fonctionnement, ou/et dans lequel une partie extérieure (52) de l'agencement de conduit (50) s'étend à l'extérieur d'un volume de canal de l'agencement de canaux (46) pouvant être traversé par le gaz respiratoire, de telle sorte qu'une partie extérieure (52) de l'agencement de conduit (50) ne peut pas être mouillée par le gaz respiratoire pendant le fonctionnement.

6. Agencement de soupape de gaz respiratoire (36) selon la revendication 5,
**caractérisé en ce que** la partie intérieure (54) de l'agencement de conduit (50) et au moins une partie de la partie extérieure (52) de l'agencement de conduit (50) sont formées entre les zones d'extrémité distale (37) de l'agencement de soupape de gaz respiratoire (36) et l'agencement de soupape (44).

7. Agencement de soupape de gaz respiratoire (36) selon la revendication 5 ou 6,
**caractérisé en ce que** l'agencement de conduit (50) traverse le boîtier (42) de l'agencement de soupape de gaz respiratoire (36) entre les zones d'extrémité distale (37) de l'agencement de soupape de gaz respiratoire (36) et l'agencement de soupape (44).

8. Agencement de soupape de gaz respiratoire (36) selon l'une des revendications précédentes,
**caractérisé en ce que** les corps de soupape (668) sont des lames de corps de soupape pour former des soupapes à lame.

9. Dispositif de ventilation (10) pour la ventilation artificielle au moins assistée d'un patient vivant, comprenant :
- une source de gaz respiratoire (12),
- un agencement de conduit respiratoire (20) pour diriger le gaz respiratoire inspiratoire de la source de gaz respiratoire (12) vers un orifice de sortie de gaz respiratoire proximal (62) du côté du patient et pour diriger le gaz respiratoire expiratoire en l'éloignant d'un orifice d'entrée de gaz respiratoire proximal (62),
- un dispositif de modification de la pression (13b) pour modifier la pression du gaz respiratoire dans l'agencement de conduit respiratoire (20),
- un dispositif de commande (14) pour faire fonctionner la source de gaz respiratoire (12) ou/et le dispositif de modification de la pression (13b), et
- un agencement de soupape de gaz respiratoire (36) selon l'une des revendications précédentes.

10. Dispositif de ventilation (10) selon la revendication 9,
**caractérisé en ce que** l'agencement de conduit respiratoire (20) comprend un tuyau multi-lumières (22), notamment un tuyau bi-lumières (22), avec un agencement de conduit (50) guidé au moins le long d'une portion longitudinale du tuyau multi-lumières (22) dans au moins une lumière.

11. Dispositif de ventilation (10) selon la revendication 10,
**caractérisé en ce que** l'agencement de conduit (50) traverse une paroi de tuyau délimitant le tuyau multi-lumières (22) vers l'environnement extérieur (U).

12. Dispositif de ventilation (10) selon l'une des revendications 10 ou 11,
**caractérisé en ce que** le tuyau multi-lumières (22) relie l'agencement de soupape de gaz respiratoire (36) à une soupape expiratoire distale (30) de manière à conduire le gaz respiratoire.

13. Dispositif de ventilation (10) selon la revendication 12,
**caractérisé en ce que** la soupape expiratoire (30) est une soupape à membrane avec une membrane de soupape (76) pouvant être soulevée d'un siège de soupape (77), dans lequel la membrane de soupape (76) peut être alimentée en gaz respiratoire expiratoire sur le côté tourné vers le tuyau multi-lumières (22) et la membrane de soupape (76) peut être alimentée en gaz respiratoire inspiratoire sur le côté opposé au tuyau multi-lumières (22).

14. Dispositif de ventilation (10) selon l'une des revendications 10 à 13,
**caractérisé en ce qu'**entre l'extrémité longitudinale proximale du tuyau multi-lumières (22) d'une part et l'ouverture proximale de sortie de gaz respiratoire (62) ou/et l'ouverture proximale d'entrée de gaz respiratoire (62) d'autre part, est disposé un agencement de capteurs de spirométrie (48) auquel est relié l'agencement de conduit (50).

15. Dispositif de ventilation (10) selon la revendication 14,
**caractérisé en ce que** l'agencement de capteurs de spirométrie (48) est un agencement de capteurs de débit (48), en particulier un agencement de capteurs de débit à pression différentielle (48), pour détecter le débit de gaz respiratoire inspiratoire ou/et expiratoire.
